# EUROPEAN PATENT APPLICATION

(11) **EP 4 825 972 A1**
(43) Date of publication of application: **21.10.2026**
(21) Application number: 24903584.1
(22) Date of filing: 04.12.2024
(51) Int. Cl.: C12N 15/55, C12N 1/15, C12N 1/19, C12N 1/21, C12N 5/10, C12N 9/20, C12N 15/63

(54) **LIPASE STABILITY IMPROVEMENT METHOD**

(30) Priority: 15.12.2023 JP 2023211839
(71) Applicant: Kao Corporation, Tokyo 103-8210 (JP)
(72) Inventor: NISHIGUCHI, Hiroki, Tokyo 103-8210 (JP); SHIBATA, Yumi, Tokyo 103-8210 (JP); HIOKI, Takahiro, Tokyo 103-8210 (JP); KAWAHARA, Akihito, Tokyo 103-8210 (JP)
(74) Representative: Vossius & Partner Patentanwälte Rechtsanwälte mbB
(86) International application number: PCT/JP2024/042947
(87) International publication number: WO 2025/126927

(57) **Abstract**

Provided is a method for improving stability of a lipase. The present invention provides a lipase complex comprising two or more polypeptide molecules, wherein the two or more polypeptide molecules are connected via a disulfide bond, and at least one polypeptide molecule of the two or more polypeptide molecules is a polypeptide which consists of an amino acid sequence having an identity of at least 70% with the amino acid sequence of SEQ ID NO: 4, 6, 8, 10, 14, 16, 18, or 20 and has lipase activity, a method for producing a lipase complex, and a method for improving stability of a lipase, comprising the step of performing lipase complexation.

## Description

### Field of the Invention

The present invention relates to a method for improving stability of a lipase.

### Background of the Invention

Lipases are useful in various uses such as a laundry detergent, a dishwashing detergent, oil processing, pulp treatment, feed, and pharmaceutical intermediate synthesis, and so on. In washing, lipases contribute to removal of stains including oil by hydrolyzing ester bonds in lipids to generate fatty acids.

Lipase for washing has been adopted for use in cleansing compositions for removing oil-based stains. Patent Literature 1 describes a method for washing oil stains by bringing a detergent including a sulfosuccinic acid alkyl ester together with a lipase into contact with oil stains and leaving it to stand without applying an external force. As described in Patent Literature 2, a cleansing composition for dishwashing is also used for washing stainless or resin sink areas, and a high concentration of a chelating agent such as citric acid may be blended therein in order to confer a function of cleaning limescale and the like attached to kitchen sink areas. A compositional feature of such a detergent is to contain a large amount of water compared to general detergents and to contain a solvent in an amount larger than that of a surfactant.

Meanwhile, modifications in amino acid sequences may be performed for functional improvement of proteins including enzymes. Non Patent Literature 1 states that a side chain of a cysteine residue present in a protein exists in different oxidative states depending on ambient pH and redox potential and oxidative state may significantly change amino acid properties in a site-specific way, which can change structure and function. In Non Patent Literature 2, introduction of a cysteine residue is avoided in protein design. Thus, the introduction of a cysteine residue might inhibit structural or functional maintenance of proteins and is therefore frequently regarded as an unfavorable mutation.

Patent Literature 1: JP-A-2021-17508
Patent Literature 2: JP-B-4776997
Non Patent Literature 1: Diego Garrido Ruiz et al. Biochemistry 2022, 61(20): 2165-2176
Non Patent Literature 2: Taihei Murakami et al. Antibodies 2022, 11(1), 10

### Summary of the Invention

The present invention relates to the following 1) to 8).
1) A lipase complex comprising two or more polypeptide molecules, wherein the two or more polypeptide molecules are connected via a disulfide bond, and at least one polypeptide molecule of the two or more polypeptide molecules is a polypeptide which consists of an amino acid sequence having an identity of at least 70% with the amino acid sequence of SEQ ID NO: 4, 6, 8, 10, 14, 16, 18, or 20 and has lipase activity.
2) A method for producing a lipase complex, comprising the step of connecting, via a disulfide bond, two or more polypeptide molecules comprising at least one molecule of a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
3) A method for improving stability of a lipase, comprising the step of connecting, via a disulfide bond, two or more polypeptide molecules comprising at least one molecule of a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
4) A polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
5) A polynucleotide encoding the polypeptide according to 4).
6) A vector or DNA fragment comprising the polynucleotide according to 5).
7) A transformed cell comprising the vector or DNA fragment according to 6).
8) A cleansing composition comprising one or both of the complex according to 1) and the polypeptide according to 4).

### Brief Description of Drawings

Figure 1 illustrates results of SDS-PAGE of a monomer fraction or a complex fraction of each lipase in the presence or absence of a reducing agent.
Figure 2 illustrates an identity matrix of the amino acid sequence of each lipase.

### Detailed Description of the Invention

All Patent Literatures, Non Patent Literatures, and other publications cited herein are incorporated by reference in their entireties.

As used herein, the term "lipase" refers to triacyl glycerollipase (EC3.1.1.3) and means an enzyme group that has lipase activity which hydrolyzes an ester bond in a lipid to generate a fatty acid. The lipase activity can be determined by measuring the rate of increase in absorbance due to release of 4-nitrophenol resulting from hydrolysis of 4-nitrophenyl butyrate. A specific procedure of the measurement of the lipase activity is described in detail in Examples mentioned later.

As used herein, the identity between amino acid sequences or nucleotide sequences is calculated by the Lipman-Pearson method (Science, 1985, 227: 1435-1441). Specifically, the identity is calculated by analysis using the homology analysis (Search homology) program of genetic information processing software GENETYX Ver. 12 by setting the Unit size to compare (ktup) to 2.

As used herein, the term "an identity of at least 70%" with respect to an amino acid sequence or nucleotide sequence refers to an identity of 70% or more, preferably 80% or more, more preferably 85% or more, further preferably 90% or more, further preferably 93% or more, further preferably 94% or more, further preferably 95% or more, further preferably 96% or more, further preferably 97% or more, further preferably 98% or more, further preferably 99% or more, and further preferably 99.5% or more.

As used herein, a "corresponding position" on an amino acid sequence or nucleotide sequence can be determined by aligning a target sequence and a reference sequence (for example, the amino acid sequence represented by SEQ ID NO: 4) so as to give the maximum homology. The alignment of amino acid sequences or nucleotide sequences can be carried out using a known algorithm, and the procedure thereof is known to those skilled in the art. For example, the alignment can be performed by using Clustal W multiple alignment program (Thompson, J.D., et al., 1994, Nucleic Acids Res., 22: 4673-4680) with the default set. Alternatively, Clustal W2 or Clustal omega, which are revised editions of Clustal W, can also be used. Clustal W, Clustal W2, and Clustal omega can be used on the website of, for example, the Clustal [www.clustal.org] operated by University College Dublin, the European Bioinformatics Institute: EBI [www.ebi.ac.uk/index.html], or the DNA data bank of Japan (DDBJ [www.ddbj.nig.ac.jp/searches-j.html]) operated by the National Institute of Genetics. A position in a target sequence aligned at any position in a reference sequence by the above-described alignment is considered to be a "position corresponding" to the position.

Those skilled in the art can further finely adjust and optimize the alignment of the amino acid sequence obtained above. The optimum alignment is preferably determined by considering the similarity of amino acid sequences, the frequency of gaps to be inserted, and so on. Here, the similarity of amino acid sequences refers to, when two amino acid sequences are aligned, the ratio (%) of the number of positions where the same or similar amino acid residues are present in both sequences to the number of the full-length amino acid residues. The similar amino acid residues mean that the amino acid residues have similar properties in the polarity or electric charge to each other and cause so-called conservative substitution among 20 amino acids constituting a protein. Groups consisting of such similar amino acid residues are well known to those skilled in the art, and examples thereof include, but not limited to, arginine and lysine or glutamine; glutamic acid and aspartic acid or glutamine; serine and threonine or alanine; glutamine and asparagine or arginine; and leucine and isoleucine.

As used herein, the term "amino acid residue" means 20 amino acid residues constituting a protein: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

As used herein, an amino acid position and variant are designated using the accepted IUPAC one-letter amino acid abbreviation as follows.

An amino acid at a predetermined position is designated by [amino acid, position]. For example, leucine at position 208 is shown as "L208".

"Substitution" of an amino acid is designated by [original amino acid, position, substituted amino acid]. For example, substitution of leucine at position 208 with cysteine is designated as "L208C".

A variant including a plurality of modifications is indicated by an addition symbol ("+"). For example, "L208C + N272C" represents substitution of leucine at position 208 with cysteine and substitution of asparagine at position 272 with cysteine.

If different modifications may be introduced at one position, the different modifications are separated by slashes ("/"). For example, "G22C/M" represents substitution of glycine at position 22 with cysteine or methionine.

As used herein, the term "operable linkage" between a regulatory region, such as a promoter, and a gene indicates that a gene and a regulatory region are linked such that the gene can be expressed under the control of the regulatory region. The procedure of "operable linkage" between a gene and a regulatory region is known to those skilled in the art.

As used herein, the terms "upstream" and "downstream" with respect to a gene refer to the upstream and downstream of the gene in the transcription direction. For example, "a gene placed downstream of a promoter" means that the gene is present on the 3' side of the promoter in a DNA sense strand, and upstream of a gene means a region on the 5' side of the gene in a DNA sense strand.

As used herein, the "parent" polypeptide of a given variant polypeptide refers to a polypeptide that brings about the variant polypeptide by a predetermined mutation of an amino acid residue. In other words, the "parent" polypeptide is a polypeptide before the mutation is introduced into the variant polypeptide.

As used herein, the "lipase complex" refers to an integrated form, via a disulfide bond, of two or more polypeptide molecules containing at least one molecule of a polypeptide (lipase) having lipase activity. The individual polypeptides constituting the lipase complex are not particularly limited as long as at least one polypeptide molecule is a lipase, regardless of whether the other polypeptide(s) may or may not have lipase activity. The individual polypeptides may be of the same type or different types and are preferably lipases of the same type or different types, more preferably lipases of the same type. The number of polypeptides constituting the complex is preferably 2, though it is not particularly limited. Accordingly, the lipase complex is preferably a dimer of two lipase molecules of the same type or different types, i.e., a lipase dimer, more preferably a lipase dimer of two lipase molecules of the same type. Hereinafter, the lipase complex is also simply referred to as a complex.

Although the stability of a lipase blended in a cleansing composition which contains a large amount of water compared to general detergents and contains a solvent in an amount larger than that of a surfactant had been unknown in such severe environments for enzymes, the present inventors have revealed as a result of studies that the stability is significantly reduced compared to the case where a lipase is blended in general detergents. Accordingly, the present invention relates to providing a method for improving stability of a lipase and a lipase which exhibits excellent stability.

The present inventors substituted an amino acid residue at a specific position in the amino acid sequence of a parent lipase with cysteine and consequently found that the obtained lipase variant can form a complex containing the lipase variant and, particularly, in a complex form, has improved stability in the presence of a large amount of water and/or a larger amount of a solvent than that of a surfactant compared to the parent lipase, i.e., stability of a lipase can be improved by lipase complexation.

According to the present invention, stability of a lipase can be improved by lipase complexation. A polypeptide which is capable of forming a lipase complex and has lipase activity exhibits excellent stability, particularly, in a complex form, and can be suitably blended even in a cleansing composition containing a large amount of water and/or a larger amount of a solvent than that of a surfactant.

### <1. Polypeptide having lipase activity>

The polypeptide which is capable of forming a lipase complex and has lipase activity according to the present invention (referred to as the "polypeptide of the present invention") is a polypeptide which consists of an amino acid sequence having an identity of at least 70% with the amino acid sequence of SEQ ID NO: 4, 6, 8, 10, 14, 16, 18, or 20 and has lipase activity. The polypeptide of the present invention has cysteine involved in a disulfide bond in the amino acid sequence. The polypeptide of the present invention is preferably a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.

In one embodiment, the polypeptide of the present invention is a natural (wild-type) polypeptide which consists of an amino acid sequence having an identity of at least 70% with the amino acid sequence of SEQ ID NO: 4, 6, 8, 10, 14, 16, 18, or 20 and has lipase activity. The natural polypeptide can have favorable stability in a complex form formed via a disulfide bond. In another embodiment, the polypeptide of the present invention is a variant polypeptide which consists of an amino acid sequence having an identity of at least 70% with the amino acid sequence of SEQ ID NO: 4, 6, 8, 10, 14, 16, 18, or 20 and has lipase activity (the variant polypeptide among the polypeptides of the present invention is also particularly referred to as the "variant polypeptide of the present invention"). The variant polypeptide can have favorable stability in a complex form formed via a disulfide bond. Here, the stability more specifically refers to stability in the presence of a large amount of water and/or a larger amount of a solvent than that of a surfactant.

In one preferable embodiment, the polypeptide of the present invention is a natural (wild-type) polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity. Cysteine at the predetermined position contributes to lipase complex formation and lipase stability. Accordingly, the natural polypeptide can form a complex, has favorable stability in a monomeric form, and can have favorable stability, particularly, in a complex form. In another preferable embodiment, the polypeptide of the present invention is a variant polypeptide which consists of an amino acid sequence obtained from the amino acid sequence of a parent lipase by substitution with cysteine of an amino acid residue at one or more positions selected from the group consisting of positions corresponding to positions 208 and 272 numbered according to SEQ ID NO: 4, and has lipase activity, and the variant polypeptide consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered based on SEQ ID NO: 4, and has lipase activity. The substitution of the amino acid residue at the predetermined position with cysteine is a modification that contributes to lipase complex formation and improves lipase stability. Accordingly, a polypeptide after amino acid residue substitution at the predetermined position in the amino acid sequence of the parent lipase, i.e., a variant polypeptide which consists of an amino acid sequence after the amino acid residue substitution and has lipase activity, can form a complex, has improved stability in a monomeric form compared to the parent lipase, and has far improved stability, particularly, in a complex form compared to the parent lipase. In general, introduction of cysteine into proteins might inhibit structural or functional maintenance of the proteins and is therefore frequently regarded as an unfavorable mutation. Moreover, the influence of lipase complexation on stability has not been known. Thus, it is totally unexpected that stability is improved by lipase complexation, particularly, lipase complexation based on substitution of an amino acid residue at a specific position in the amino acid sequence of a lipase with cysteine. Here, the stability more specifically refers to stability in the presence of a large amount of water and/or a larger amount of a solvent than that of a surfactant.

In the present invention, the parent lipase is a polypeptide which consists of an amino acid sequence having an identity of at least 70% with the amino acid sequence of SEQ ID NO: 4, 6, 8, 10, 14, 16, 18, or 20 and has lipase activity. The parent lipase may be a natural (wild-type) polypeptide or its variant.

An example of the parent lipase includes a polypeptide which consists of an amino acid sequence having an identity of at least 70% with the amino acid sequence of SEQ ID NO: 4 and has lipase activity. Here, the lipase consisting of the amino acid sequence of SEQ ID NO: 4 is a lipase which has amino acid residue substitutions I44T, L120S, S130A, A134T, A135V, L136M, and S137N in an amino acid sequence constituting lipase CnLip (NCBI Accession No. WP_061278013.1) derived from *Cedecea neteri* (CnLipA excellent in detergency described in Japanese Patent Application No. 2023-025141) and further has amino acid residue substitutions F16L, I47L, A91V, E97D, and A149E, and is CnLipAm shown in Examples described later. Both the CnLip and the CnLipA are capable of serving as the parent lipase in the present invention. The parent lipase which is the polypeptide which consists of an amino acid sequence having an identity of at least 70% with the amino acid sequence of SEQ ID NO: 4 and has lipase activity. The parent lipase preferably has leucine at a position corresponding to position 208 numbered according to SEQ ID NO: 4 and/or has asparagine at a position corresponding to position 272 numbered according to SEQ ID NO: 4, and more preferably has leucine at a position corresponding to position 208 and asparagine at a position corresponding to position 272 numbered according to SEQ ID NO: 4.

Another example of the parent lipase includes a polypeptide which consists of an amino acid sequence having an identity of at least 70% with the amino acid sequence of SEQ ID NO: 6 and has lipase activity. Here, the lipase consisting of the amino acid sequence of SEQ ID NO: 6 is a lipase which has an amino acid residue substitution E118D in an amino acid sequence constituting lipase SspLip (NCBI Accession No. WP_025122441.1) derived from *Serratia* sp. (variant SspLip_m excellent in productivity described in Japanese Patent Application No. 2022-149382). The parent lipase which is the polypeptide which consists of an amino acid sequence having an identity of at least 70% with the amino acid sequence of SEQ ID NO: 6 and has lipase activity preferably has leucine at a position corresponding to position 208 numbered according to SEQ ID NO: 4 and/or has proline at a position corresponding to position 272 numbered according to SEQ ID NO: 4, and more preferably has leucine at a position corresponding to position 208 and proline at a position corresponding to position 272 numbered according to SEQ ID NO: 4. The positions corresponding to positions 208 and 272 numbered according to SEQ ID NO: 4 are positions 210 and 274, respectively, in the amino acid sequence of SEQ ID NO: 6.

An alternative example of the parent lipase includes a polypeptide which consists of an amino acid sequence having an identity of at least 70% with the amino acid sequence of SEQ ID NO: 8 and has lipase activity. Here, the lipase consisting of the amino acid sequence of SEQ ID NO: 8 is a lipase which has an amino acid residue substitution I222G in an amino acid sequence constituting lipase EtLip (NCBI Accession No. WP_115457195.1) derived from *Enterobacillus tribolii* (variant EtLip_m excellent in productivity described in Japanese Patent Application No. 2022-149382). The parent lipase which is the polypeptide which consists of an amino acid sequence having an identity of at least 70% with the amino acid sequence of SEQ ID NO: 8 and has lipase activity preferably has phenylalanine at a position corresponding to position 208 numbered according to SEQ ID NO: 4 and/or has threonine at a position corresponding to position 272 numbered according to SEQ ID NO: 4, and more preferably has phenylalanine at a position corresponding to position 208 and threonine at a position corresponding to position 272 numbered according to SEQ ID NO: 4. The positions corresponding to positions 208 and 272 numbered according to SEQ ID NO: 4 are positions 210 and 274, respectively, in the amino acid sequence of SEQ ID NO: 8.

An alternative example of the parent lipase includes a polypeptide which consists of an amino acid sequence having an identity of at least 70% with the amino acid sequence of SEQ ID NO: 10 and has lipase activity. Here, the lipase consisting of the amino acid sequence of SEQ ID NO: 10 is a lipase which has amino acid residue substitutions I45T, L121S, S131A, A135T, A136V, and S138N in an amino acid sequence constituting lipase CspLip (NCBI Accession No. WP_016537805.1) derived from *Cedecea* sp. (CspLipA excellent in detergency described in Japanese Patent Application No. 2023-025141). The parent lipase which is the polypeptide which consists of an amino acid sequence having an identity of at least 70% with the amino acid sequence of SEQ ID NO: 10 and has lipase activity preferably has leucine at a position corresponding to position 208 numbered according to SEQ ID NO: 4 and/or has asparagine at a position corresponding to position 272 numbered according to SEQ ID NO: 4, and more preferably has leucine at a position corresponding to position 208 and asparagine at a position corresponding to position 272 numbered according to SEQ ID NO: 4. The positions corresponding to positions 208 and 272 numbered according to SEQ ID NO: 4 are positions 209 and 273, respectively, in the amino acid sequence of SEQ ID NO: 10.

An alternative example of the parent lipase includes a polypeptide which consists of an amino acid sequence having an identity of at least 70% with the amino acid sequence of SEQ ID NO: 14 and has lipase activity. Here, the lipase consisting of the amino acid sequence of SEQ ID NO: 14 is KAL (JP-A-2023-156914). The parent lipase which is the polypeptide which consists of an amino acid sequence having an identity of at least 70% with the amino acid sequence of SEQ ID NO: 14 and has lipase activity preferably has leucine at a position corresponding to position 208 numbered according to SEQ ID NO: 4 and/or has serine at a position corresponding to position 272 numbered according to SEQ ID NO: 4, and more preferably has leucine at a position corresponding to position 208 and serine at a position corresponding to position 272 numbered according to SEQ ID NO: 4. The positions corresponding to positions 208 and 272 numbered according to SEQ ID NO: 4 are positions 210 and 274, respectively, in the amino acid sequence of SEQ ID NO: 14.

An alternative example of the parent lipase includes a polypeptide which consists of an amino acid sequence having an identity of at least 70% with the amino acid sequence of SEQ ID NO: 16 and has lipase activity. Here, the lipase consisting of the amino acid sequence of SEQ ID NO: 16 is ancestral lipase AncLip10 (JP-A-2022-66571). Here, the ancestral lipase refers to a lipase consisting of an amino acid sequence which is derived from the existing sequences of lipases of organisms derived from a common ancestor on the basis of a rooted phylogenetic tree, that indicates protein evolution, and which is presumed to be carried by the common ancestor. The amino acid sequence of the ancestral lipase can be determined by ancestral sequence reconstruction (ASR) from the existing sequence of a lipase. The parent lipase which is the polypeptide which consists of an amino acid sequence having an identity of at least 70% with the amino acid sequence of SEQ ID NO: 16 and has lipase activity preferably has leucine at a position corresponding to position 208 numbered according to SEQ ID NO: 4 and/or has threonine at a position corresponding to position 272 numbered according to SEQ ID NO: 4, and more preferably has leucine at a position corresponding to position 208 and threonine at a position corresponding to position 272 numbered according to SEQ ID NO: 4. The positions corresponding to positions 208 and 272 numbered according to SEQ ID NO: 4 are positions 209 and 273, respectively, in the amino acid sequence of SEQ ID NO: 16.

An alternative example of the parent lipase includes a polypeptide which consists of an amino acid sequence having an identity of at least 70% with the amino acid sequence of SEQ ID NO: 18 and has lipase activity. Here, the lipase consisting of the amino acid sequence of SEQ ID NO: 18 is a lipase (NCBI Accession No. WP_045783583.1) derived from *Enterobacteriaceae.* The parent lipase which is the polypeptide which consists of an amino acid sequence having an identity of at least 70% with the amino acid sequence of SEQ ID NO: 18 and has lipase activity preferably has leucine at a position corresponding to position 208 numbered according to SEQ ID NO: 4 and/or has serine at a position corresponding to position 272 numbered according to SEQ ID NO: 4, and more preferably has leucine at a position corresponding to position 208 and serine at a position corresponding to position 272 numbered according to SEQ ID NO: 4. The positions corresponding to positions 208 and 272 numbered according to SEQ ID NO: 4 are positions 208 and 272, respectively, in the amino acid sequence of SEQ ID NO: 18.

An alternative example of the parent lipase includes a polypeptide which consists of an amino acid sequence having an identity of at least 70% with the amino acid sequence of SEQ ID NO: 20 and has lipase activity. Here, the lipase consisting of the amino acid sequence of SEQ ID NO: 20 is lipase (NCBI Accession No. WP_037407093.1) derived from *Chania multitudinisentens.* The parent lipase which is the polypeptide which consists of an amino acid sequence having an identity of at least 70% with the amino acid sequence of SEQ ID NO: 20 and has lipase activity preferably has isoleucine at a position corresponding to position 208 numbered according to SEQ ID NO: 4 and/or has glycine at a position corresponding to position 272 numbered according to SEQ ID NO: 4, and more preferably has isoleucine at a position corresponding to position 208 and glycine at a position corresponding to position 272 numbered according to SEQ ID NO: 4. The positions corresponding to positions 208 and 272 numbered according to SEQ ID NO: 4 are positions 208 and 272, respectively, in the amino acid sequence of SEQ ID NO: 20.

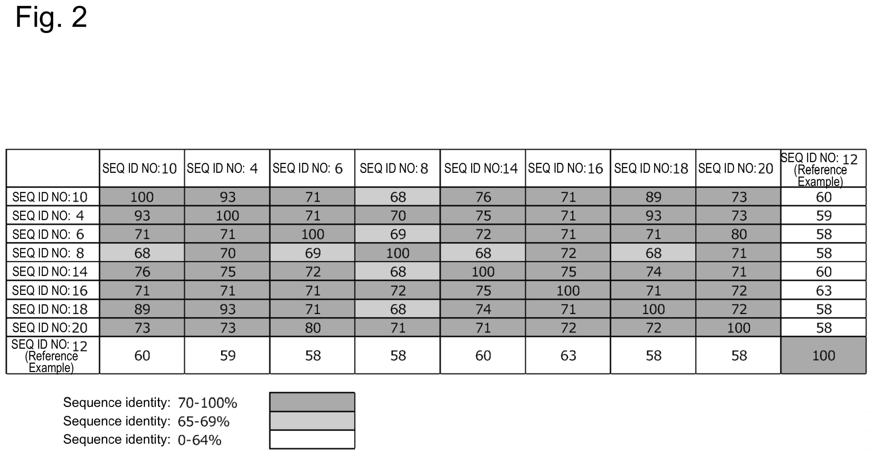

The amino acid sequence of SEQ ID NO: 4, 6, 8, 10, 14, 16, 18, or 20 has an identity of at least 70% with at least one other amino acid sequence among the amino acid sequences of SEQ ID NOs: 4, 6, 8, 10, 14, 16, 18, and 20, except for itself (Figure 2). For example, all the amino acid sequences of SEQ ID NOs: 6, 8, 10, 14, 16, 18, and 20 have an identity of at least 70% with the amino acid sequence of SEQ ID NO: 4. On the other hand, all the amino acid sequences of SEQ ID NOs: 4, 6, 8, 10, 14, 16, 18, and 20 do not have an identity of at least 70% with the amino acid sequence (SEQ ID NO: 12) of lipase PvLip of Reference Example in Examples described later.

The polypeptide of the present invention is preferably a polypeptide which consists of an amino acid sequence having an identity of at least 70% with the amino acid sequence of SEQ ID NO: 4 and having cysteine as an amino acid residue at one or more positions selected from the group consisting of positions corresponding to positions 208 and 272 numbered according to SEQ ID NO: 4, and has lipase activity, and a polypeptide which consists of an amino acid sequence having an identity of at least 70% with the amino acid sequence of SEQ ID NO: 6, 8, 10, 14, 16, 18, or 20 and having cysteine as an amino acid residue at a position corresponding to position 208 numbered according to SEQ ID NO: 4, and has lipase activity, more preferably a polypeptide which consists of an amino acid sequence having an identity of at least 70% with the amino acid sequence of SEQ ID NO: 4 and having cysteine as an amino acid residue at one or more positions selected from the group consisting of positions corresponding to positions 208 and 272 numbered according to SEQ ID NO: 4, and has lipase activity, and a polypeptide which consists of an amino acid sequence having an identity of at least 70% with the amino acid sequence of SEQ ID NO: 6, 8, or 10 and having cysteine as an amino acid residue at a position corresponding to position 208 numbered according to SEQ ID NO: **4,** and has lipase activity, from the viewpoint of stability.

The polypeptide of the present invention may have one or more amino acid residues, preferably one amino acid residue, selected from the group consisting of phenylalanine at a position corresponding to position 16, cysteine or methionine at a position corresponding to position 22, phenylalanine at a position corresponding to position 29, isoleucine or valine at a position corresponding to position 50, alanine or glutamine at a position corresponding to position 120, isoleucine or threonine at a position corresponding to position 123, glutamic acid at a position corresponding to position 126, phenylalanine at a position corresponding to position 142, alanine at a position corresponding to position 156, lysine at a position corresponding to position 181, glutamic acid at a position corresponding to position 186, glutamine at a position corresponding to position 188, valine at a position corresponding to position 191, histidine at a position corresponding to position 220, histidine at a position corresponding to position 221, glutamine at a position corresponding to position 224, threonine at a position corresponding to position 233, asparagine at a position corresponding to position 256, alanine, cysteine, or leucine at a position corresponding to position 260, isoleucine at a position corresponding to position 269, and glutamine at a position corresponding to position 274 numbered according to SEQ ID NO: 4, without inhibiting complex formation.

### <2. Polynucleotide encoding polypeptide of present invention>

The polypeptide of the present invention can be produced by expression of the polypeptide from a polynucleotide encoding the polypeptide. The polynucleotide can be prepared from a microorganism that produces the lipase of interest by extracting genomic DNA by a usual method or extracting RNA and synthesizing cDNA therefrom by reverse transcription. Alternatively, a corresponding nucleotide sequence chemically synthesized based on the amino acid sequence of the lipase of interest may be used as the polynucleotide encoding the polypeptide of the present invention.

Alternatively, the polypeptide of the present invention can be produced using various mutagenesis techniques known in the art. For example, the polypeptide can be produced by mutating a polynucleotide encoding the amino acid residue to be substituted in a parent lipase gene (reference lipase gene) encoding the reference amino acid sequence into a polynucleotide encoding an amino acid residue after mutation, and further expressing a variant polypeptide from the variant gene.

In the present invention, as the method for mutating an amino acid residue of a parent lipase, various mutagenesis techniques known in the art can be used. For example, a polynucleotide encoding the variant polypeptide of the present invention can be obtained by mutating a nucleotide sequence encoding an amino acid residue to be mutated into a nucleotide sequence encoding the amino acid residue after mutation in a polynucleotide encoding the amino acid sequence of a parent lipase (hereinafter, also referred to as parent gene).

Basically, introduction of a desired mutation into a parent gene can be performed using various site-directed mutagenesis methods known to those skilled in the art. The site-directed mutagenesis can be performed by an arbitrary method, such as an inverse PCR method or an annealing method. Commercially available site-directed mutagenesis kits (for example, QuickChange II Site-Directed Mutagenesis Kit and QuickChange Multi Site-Directed Mutagenesis Kit of Stratagene) can also be used.

Site-directed mutagenesis into a parent gene can be most generally performed using a mutation primer including a nucleotide mutation to be introduced. The mutation primer may be designed so as to anneal with a region including a nucleotide sequence that encodes an amino acid residue to be mutated in a parent gene and to include a nucleotide sequence having a nucleotide sequence (codon) that encodes an amino acid residue after the mutation instead of the nucleotide sequence (codon) encoding the amino acid residue to be mutated. Nucleotide sequences (codons) encoding amino acid residues before and after mutation can be appropriately recognized and selected by those skilled in the art based on ordinary textbooks and the like. Alternatively, as site-directed mutagenesis, a method of linking DNA fragments into one by SOE (splicing by overlap extension)-PCR (Gene, 1989, 77(1): 61-68), wherein the DNA fragments are separately obtained by amplifying both the upstream and downstream sides of a mutation site with two complementary primers including the nucleotide mutation to be introduced can be used.

A template DNA including a parent gene can be prepared from a microorganism that produces the above-described parent lipase by extracting genomic DNA by a usual method or extracting RNA and synthesizing cDNA therefrom by reverse transcription. Alternatively, a corresponding nucleotide sequence chemically synthesized based on the amino acid sequence of a parent lipase may be used as a template DNA. DNA sequences including nucleotide sequences encoding lipases consisting of amino acid sequences of SEQ ID NOs: 4, 6, 8, 10, 14, 16, 18, and 20 are represented by SEQ ID NOs: 3, 5, 7, 9, 13, 15, 17, and 19, respectively.

A mutation primer can be produced by a known oligonucleotide synthesis method such as a phosphoramidite method (Nucleic Acids Research, 1989, 17: 7059-7071). Such primer synthesis can also be carried out using, for example, a commercially available oligonucleotide synthesis apparatus (for example, manufactured by ABI Inc.). A polynucleotide encoding a variant polypeptide with a desired mutation of the present invention can be obtained using a primer set including the mutation primer and performing site-directed mutagenesis as described above using a parent gene as a template DNA.

The polynucleotide encoding the polypeptide of the present invention can include a single-stranded or double-stranded DNA, cDNA, RNA, or another artificial nucleic acid. The DNA, cDNA, and RNA may be chemically synthesized. The polynucleotide may include a nucleotide sequence of an untranslated region (UTR), in addition to an open reading frame (ORF). The polynucleotide may be codon-optimized in accordance with the type of a transformant for producing the polypeptide of the present invention. Information on codons to be used by various organisms is available from the Codon Usage Database ([www.kazusa.or.jp/codon/]) .

### <3. Vector or DNA fragment>

The obtained polynucleotide encoding the polypeptide of the present invention can be incorporated into a vector. The type of the vector containing the polynucleotide is not particularly limited and may be any vector such as a plasmid, phage, phagemid, cosmid, virus, YAC vector, and shuttle vector. The vector is, but not limited to, preferably, a vector capable of being amplified in bacteria, preferably, in *Bacillus* bacteria (for example, *Bacillus subtilis* or its mutant strain), more preferably, an expression vector capable of inducing expression of a transgene in *Bacillus* bacteria. In particular, a shuttle vector, which is a vector capable of replicating in both *Bacillus* bacteria and other organisms, can be suitably used for recombinant production of the polypeptide of the present invention. Preferable examples of the vector include, but not limited to, shuttle vectors, such as pHA3040SP64, pHSP64R, and pASP64 (Japanese Patent No. 3492935), pHY300PLK (expression vector capable of transforming both *Escherichia coli* and *Bacillus subtilis;* Jpn J. Genet., 1985, 60: 235-243), and pAC3 (Nucleic Acids Res., 1988, 16: 8732); and plasmid vectors that can be used for transformation of *Bacillus* bacteria, such as pUB110 (J. Bacteriol., 1978, 134: 318-329) and pTA10607 (Plasmid, 1987, 18: 8-15). In addition, plasmid vectors derived from *Escherichia coli* (e.g., pET22b(+), pBR322, pBR325, pUC57, pUC118, pUC119, pUC18, pUC19, and pBluescript) can also be used.

The above vectors can include a DNA replication initiation region or a DNA region including replication origin. Alternatively, in the above vectors, a control sequence may be operably linked to the upstream of a polynucleotide encoding the polypeptide of the present invention, wherein the control sequence is a promoter region for initiating transcription of the gene, terminator region, secretory signal region for extracellularly secreting the expressed protein, or the like.

The type of the control sequence, such as the promoter region, terminator region, and secretory signal region, is not particularly limited, and a promoter or secretory signal sequence that is usually used can be appropriately selected and used depending on the host into which the sequence is introduced. For example, suitable examples of the control sequence capable of being incorporated into a vector include a promoter of the cellulase gene of *Bacillus* sp. KSM-S237 strain and a secretory signal sequence.

Alternatively, a marker gene (e.g., a gene resistant to drugs, such as ampicillin, neomycin, kanamycin, and chloramphenicol) for selecting a host into which the vector has been appropriately introduced may be further incorporated into the vector of the present invention. Alternatively, when an auxotrophic strain is used as the host, a gene encoding an enzyme that synthesizes a required nutrient may be incorporated into a vector as a marker gene. Alternatively, when a selection medium that requires a specific metabolism for growth is used, a gene related to the metabolism may be incorporated into a vector as a marker gene. Examples of such a metabolism-related gene include an acetamidase gene for using acetamide as a nitrogen source.

The polynucleotide encoding the polypeptide of the present invention may be linked to a control sequence and a marker gene by a method known in the art such as an SOE (splicing by overlap extension)-PCR method (Gene, 1989, 77: 61-68). The procedure of introducing the linked fragments into a vector is well known in the art.

### <4. Transformed cell>

A transformed cell of the present invention can be obtained by introducing a vector including a polynucleotide encoding the polypeptide of the present invention into a host or by introducing a DNA fragment including a polynucleotide encoding the polypeptide of the present invention into the genome of a host.

Examples of the host cell include microorganisms such as bacteria and filamentous fungi. Examples of the bacteria include *Escherichia coli* and bacteria belonging to *Staphylococcus, Enterococcus, Listeria,* and *Bacillus,* and among them, preferred are *Escherichia coli* and *Bacillus* bacteria, more preferred are *Bacillus* bacteria, and further preferred are *Bacillus subtilis* (e.g., *Bacillus subtilis* Marburg No. 168 strain and its mutant strain). Examples of the *Bacillus subtilis* mutant strain include a nine protease-deficient strain KA8AX described in J. Biosci. Bioeng., 2007, 104(2): 135-143 and D8PA strain that is an eight protease-deficient strain with improved protein folding efficiency described in Biotechnol. Lett., 2011, 33(9): 1847-1852. Examples of the filamentous fungus include *Trichoderma, Aspergillus,* and *Rhizopus.*

As the method for introducing a vector into a host, a method that is usually used in the art, such as a protoplast method and an electroporation method, can be used. A desired transformant into which a vector has been introduced can be obtained by selecting a strain in which the introduction has been appropriately performed by using expression of a marker gene, auxotrophy, or the like as an indicator.

Alternatively, a fragment in which a polynucleotide encoding the polypeptide of the present invention, a control sequence, and a marker gene are linked can also be directly introduced into the genome of a host. For example, a DNA fragment in which a sequence complementary to the genome of a host has been added to both ends of the linked fragment is constructed by an SOE-PCR method or the like and is introduced into a host to cause homologous recombination between the host genome and the DNA fragment. Consequently, the polynucleotide encoding the polypeptide of the present invention is introduced into the genome of the host.

When the thus-obtained transformant into which a polynucleotide encoding the polypeptide of the present invention or a vector including it has been introduced is cultured in an appropriate medium, a gene encoding the protein on the vector is expressed to generate the polypeptide of the present invention. The medium that is used for culturing the transformant can be appropriately selected depending on the type of the microorganism of the transformant by those skilled in the art.

Alternatively, the polypeptide of the present invention may be expressed from a polynucleotide encoding the polypeptide of the present invention or its transcriptional product using a cell-free translation system. The "cell-free translation system" is an in vitro transcription translation system or in vitro translation system constructed by adding a reagent, such as amino acid, necessary for translation of a protein to a suspension obtained by mechanically disrupting the cells as the host.

### <5. Lipase complex>

The polypeptide of the present invention can form a lipase complex by connection via a disulfide bond, preferably by connection via a disulfide bond under a non-reducing condition. The lipase complex formed by the polypeptide of the present invention is a complex containing at least one molecule of the polypeptide of the present invention. Examples of the complex include a complex in which the polypeptides of the present invention of the same type or different types are integrated via a disulfide bond, and a complex in which the polypeptide of the present invention is integrated via a disulfide bond with a component other than the polypeptide of the present invention. The component other than the polypeptide of the present invention constituting the complex is a polypeptide capable of forming a disulfide bond with cysteine of the polypeptide of the present invention. Examples thereof include a polypeptide containing cysteine which can be formed by a host (e.g., *Bacillus subtilis)* when the polypeptide of the present invention is expressed. The lipase complex formed by the polypeptide of the present invention is preferably a complex containing two or more polypeptide molecules containing at least one molecule of the polypeptide of the present invention, more preferably a lipase dimer of two molecules of the polypeptides of the present invention of the same type or different types, further more preferably a lipase dimer of two molecules of the polypeptides of the present invention of the same type. Specifically, the lipase complex formed by the polypeptide of the present invention is preferably a complex containing two or more polypeptide molecules, wherein the two or more polypeptide molecules are connected via a disulfide bond, and at least one polypeptide molecule of the two or more polypeptide molecules is a polypeptide which consists of an amino acid sequence having an identity of at least 70% with the amino acid sequence of SEQ ID NO: 4, 6, 8, 10, 14, 16, 18, or 20 and has lipase activity, more preferably a complex containing two or more polypeptide molecules, wherein the two or more polypeptide molecules are connected via a disulfide bond, and at least one polypeptide molecule of the two or more polypeptide molecules is a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity, further more preferably a lipase dimer consisting of two polypeptide molecules, wherein the two polypeptide molecules are connected via a disulfide bond, and the two polypeptide molecules are each independently a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity, further more preferably a lipase dimer consisting of two identical polypeptide molecules, wherein the two identical polypeptide molecules are connected via a disulfide bond, and the polypeptide is a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.

### <6. Method for producing lipase complex>

The present invention also provides a method for producing a lipase complex. The method for producing a lipase complex according to the present invention includes the step of connecting, via a disulfide bond, two or more polypeptide molecules containing at least one molecule of the polypeptide of the present invention. Complex formation through a disulfide bond between cysteine residues in the two or more polypeptide molecules can be performed, for example, by using the transformed cell of the present invention to express the polypeptide of the present invention under a non-reducing condition or to retain the polypeptide of the present invention under a non-reducing condition. Here, the "non-reducing condition" refers to an environment where oxidative power for forming a disulfide bond is present. Examples thereof include an environment where a reducing agent such as dithiothreitol (DTT) is not present in an amount that inhibit formation of a disulfide bond. It is generally known that the cytoplasm is maintained under a reducing condition. The polypeptide of the present invention is therefore preferably expressed by using the transformed cell of the present invention under conditions that direct the polypeptide to an oxidative environment such as the endoplasmic reticulum or the periplasm, or under conditions which cause extracellular release of the polypeptide by secretion or lysis after cultivation. Alternatively, it is preferable to use the transformed cell of the present invention to express the polypeptide of the present invention or to retain the polypeptide of the present invention in an aerated and stirred state in order to promote air oxidation. The complex formation can also be performed by using a cell-free translation system to express the polypeptide of the present invention under a non-reducing condition or to retain the polypeptide of the present invention under a non-reducing condition. It is preferable to express the polypeptide of the present invention using the cell-free translation system by the addition of chaperone such as glutathione, disulfide bond isomerase (DsbC), or protein disulfide isomerase (PDI) for reinforcement of oxidative power in the cell-free translation system. Alternatively, it is preferable to use the cell-free translation system to express the polypeptide of the present invention or to retain the polypeptide of the present invention in an aerated and stirred state in order to promote air oxidation.

The method for producing a lipase complex according to the present invention may further include, prior to the connection step described above, the step of providing the polypeptide of the present invention. In one preferable embodiment, the step includes the step of substituting, with cysteine, an amino acid residue at a specific position that contributes to polypeptide integration in the amino acid sequence of at least one parent lipase. In one more preferable embodiment, the step includes the step of substituting, with cysteine, an amino acid residue at one or more positions selected from the group consisting of positions corresponding to positions 208 and 272 numbered based on SEQ ID NO: 4 in at least one polypeptide which consists of an amino acid sequence having an identity of at least 70% with the amino acid sequence of SEQ ID NO: 4, 6, 8, 10, 14, 16, 18, or 20 and has lipase activity.

The substitution of the amino acid residue at a predetermined position with cysteine is a modification which contributes to lipase complex formation. The position of amino acid residue substitution is preferably one or more positions selected from the group consisting of positions corresponding to positions 208 and 272 numbered according to SEQ ID NO: 4 in a polypeptide which consists of an amino acid sequence having an identity of at least 70% with the amino acid sequence of SEQ ID NO: 4 and has lipase activity, and position 208 numbered according to SEQ ID NO: 4 in a polypeptide which consists of an amino acid sequence having an identity of at least 70% with the amino acid sequence of SEQ ID NO: 6, 8, 10, 14, 16, 18, or 20 and has lipase activity, from the viewpoint of improvement in stability.

In the step of providing the polypeptide of the present invention, an amino acid residue at one or more positions, preferably one position, selected from the group consisting of positions corresponding to positions 16, 22, 29, 50, 120, 123, 126, 142, 156, 181, 186, 188, 191, 220, 221, 224, 233, 256, 260, 269, and 274 numbered according to SEQ ID NO: 4 may be substituted in addition to the amino acid residue substitution described above, as long as complex formation is not inhibited. Examples of the preferable mode of each substitution include substitution of an amino acid residue at a position corresponding to position 16 with phenylalanine, substitution of an amino acid residue at a position corresponding to position 22 with cysteine or methionine, substitution of an amino acid residue at a position corresponding to position 29 with phenylalanine, substitution of an amino acid residue at a position corresponding to position 50 with isoleucine or valine, substitution of an amino acid residue at a position corresponding to position 120 with alanine or glutamine, substitution of an amino acid residue at a position corresponding to position 123 with isoleucine or threonine, substitution of an amino acid residue at a position corresponding to position 126 with glutamic acid, substitution of an amino acid residue at a position corresponding to position 142 with phenylalanine, substitution of an amino acid residue at a position corresponding to position 156 with alanine, substitution of an amino acid residue at a position corresponding to position 181 with lysine, substitution of an amino acid residue at a position corresponding to position 186 with glutamic acid, substitution of an amino acid residue at a position corresponding to position 188 with glutamine, substitution of an amino acid residue at a position corresponding to position 191 with valine, substitution of an amino acid residue at a position corresponding to position 220 with histidine, substitution of an amino acid residue at a position corresponding to position 221 with histidine, substitution of an amino acid residue at a position corresponding to position 224 with glutamine, substitution of an amino acid residue at a position corresponding to position 233 with threonine, substitution of an amino acid residue at a position corresponding to position 256 with asparagine, substitution of an amino acid residue at a position corresponding to position 260 with alanine, cysteine, or leucine, substitution of an amino acid residue at a position corresponding to position 269 with isoleucine, and substitution of an amino acid residue at a position corresponding to position 274 with glutamine numbered according to SEQ ID NO: 4. Any number of conservative amino acid substitutions may be performed as long as complex formation is not inhibited.

The polypeptide of the present invention can be isolated or purified in a monomeric or complex form by using general methods that are used in protein purification, for example centrifugation, ammonium sulfate precipitation, gel chromatography, ion exchange chromatography, and affinity chromatography, singly or in appropriate combination. The protein collected from the culture may be further purified by a known method.

### <7. Method for improving stability of lipase>

The polypeptide of the present invention has favorable detergency and has improved stability in a complex form compared to a monomeric form, more specifically, improved stability in a complex form in the presence of a large amount of water and/or a larger amount of a solvent than that of a surfactant compared to a monomeric form.

The "stability" means an ability to maintain lipase activity. The "improved stability in a complex form compared to a monomeric form" means an improved ability of the complex to maintain lipase activity compared to the monomer. The "improved stability in a complex form in the presence of a large amount of water and/or a larger amount of a solvent than that of a surfactant compared to a monomeric form" means an improved ability of the complex to maintain lipase activity in the presence of a large amount of water and/or a larger amount of a solvent than that of a surfactant compared to the monomer.

The variant polypeptide of the present invention has improved stability in a monomeric form compared to the parent lipase and further has far improved stability in a complex form compared to the parent lipase, more specifically, improved stability in the presence of a large amount of water and/or a larger amount of a solvent than that of a surfactant, without impairing detergency of the parent lipase.

The "stability" means an ability to maintain lipase activity. The "improved stability compared to the parent lipase" means an improved ability to maintain lipase activity compared to the parent lipase. The "improved stability in the presence of a large amount of water and/or a larger amount of a solvent than that of a surfactant compared to the parent lipase" means an improved ability to maintain lipase activity in the presence of a large amount of water and/or a larger amount of a solvent than that of a surfactant compared to the parent lipase.

Here, the stability in the presence of a large amount of water means an ability to maintain lipase activity when the lipase is preserved in a cleansing composition having a water content of from 60 to 99 mass%. The stability in the presence of a larger amount of a solvent than that of a surfactant means an ability to maintain lipase activity in a cleansing composition having a larger content of a solvent than that of a surfactant or in a washing solution having a larger content of a solvent than that of a surfactant, prepared by dissolving or diluting the cleansing composition in or with water.

Examples of the surfactant and the solvent include surfactants and solvents mentioned later.

The stability of the lipase can be evaluated using a method well known in the art. The stability of the lipase can be evaluated, for example, by adding the lipase to an appropriate cleansing composition or a washing solution prepared by dissolving or diluting the cleansing composition in or with water, measuring lipase activity before and after treatment for a predetermined time, and calculating a half-life of the activity on the basis of a deactivation rate per unit time by the treatment with the activity value of the sample before the treatment as initial activity. A value obtained by dividing the half-life of the variant polypeptide by the half-life of the parent lipase indicates relative stability of the variant polypeptide. When this value exceeds 1, the variant polypeptide is evaluated as having improved stability compared to the parent lipase. Examples of the washing solution include, but not limited to, a model washing solution having the composition shown in Table 2 in Examples described later.

The stability of the lipase complex (lipase dimer) containing the polypeptide of the present invention can be preferably 1.3 or more, more preferably 1.5 or more, in terms of relative stability to a single substance of the polypeptide of the present invention treated at 45°C for 1 hour. The stability of the variant polypeptide of the present invention can be preferably 1.5 or more, more preferably 1.7 or more, in terms of relative stability to the parent lipase treated at 45°C for 1 hour. The stability of the lipase complex (lipase dimer) containing the variant polypeptide of the present invention can be preferably 2 or more, more preferably 3 or more, in terms of relative stability to the parent lipase treated at 45°C for 1 hour.

The present invention also provides a method for improving stability of a lipase. The method for improving stability of a lipase according to the present invention includes the step of performing lipase complexation. The lipase complexation is the step of preparing a lipase complex by integrating two or more polypeptide molecules containing at least one molecule of a polypeptide having lipase activity via a disulfide bond between cysteine residues in the two or more polypeptide molecules. Specifically, the method for improving stability of a lipase according to the present invention includes the step of connecting, via a disulfide bond, two or more polypeptide molecules containing at least one molecule of the polypeptide of the present invention. Complex formation through a disulfide bond between cysteine residues in the two or more polypeptide molecules can be performed, for example, by using the transformed cell of the present invention to express the polypeptide of the present invention under a non-reducing condition or to retain the polypeptide of the present invention under a non-reducing condition. The non-reducing condition and its preferable embodiments are as described above.

The method for improving stability of a lipase according to the present invention may further include, prior to the connection step described above, the step of providing the polypeptide of the present invention. In one preferable embodiment, the step includes the step of substituting, with cysteine, an amino acid residue at a specific position that contributes to polypeptide integration in the amino acid sequence of at least one parent lipase. In one more preferable embodiment, the step includes the step of substituting, with cysteine, an amino acid residue at one or more positions selected from the group consisting of positions corresponding to positions 208 and 272 numbered based on SEQ ID NO: 4 in at least one polypeptide which consists of an amino acid sequence having an identity of at least 70% with the amino acid sequence of SEQ ID NO: 4, 6, 8, 10, 14, 16, 18, or 20 and has lipase activity.

The substitution of the amino acid residue at a predetermined position with cysteine is a modification which contributes to lipase complex formation and is for improving lipase stability. Accordingly, a polypeptide after amino acid residue substitution at the predetermined position in the amino acid sequence of the parent lipase, i.e., a variant polypeptide which consists of an amino acid sequence after the amino acid residue substitution and has lipase activity, can form a complex, has improved stability in a monomeric form compared to the parent lipase, and has far improved stability, particularly, in a complex form compared to the parent lipase. The position of amino acid residue substitution is preferably one or more positions selected from the group consisting of positions corresponding to positions 208 and 272 numbered according to SEQ ID NO: 4 in a polypeptide which consists of an amino acid sequence having an identity of at least 70% with the amino acid sequence of SEQ ID NO: 4 and has lipase activity, and position 208 numbered according to SEQ ID NO: 4 in a polypeptide which consists of an amino acid sequence having an identity of at least 70% with the amino acid sequence of SEQ ID NO: 6, 8, 10, 14, 16, 18, or 20 and has lipase activity, from the viewpoint of improvement in stability.

In the step of providing the polypeptide of the present invention, an amino acid residue at one or more positions, preferably one position, selected from the group consisting of positions corresponding to positions 16, 22, 29, 50, 120, 123, 126, 142, 156, 181, 186, 188, 191, 220, 221, 224, 233, 256, 260, 269, and 274 numbered according to SEQ ID NO: 4 may be substituted in addition to the amino acid residue substitution described above, as long as complex formation is not inhibited. Examples of the preferable mode of each substitution include substitution of an amino acid residue at a position corresponding to position 16 with phenylalanine, substitution of an amino acid residue at a position corresponding to position 22 with cysteine or methionine, substitution of an amino acid residue at a position corresponding to position 29 with phenylalanine, substitution of an amino acid residue at a position corresponding to position 50 with isoleucine or valine, substitution of an amino acid residue at a position corresponding to position 120 with alanine or glutamine, substitution of an amino acid residue at a position corresponding to position 123 with isoleucine or threonine, substitution of an amino acid residue at a position corresponding to position 126 with glutamic acid, substitution of an amino acid residue at a position corresponding to position 142 with phenylalanine, substitution of an amino acid residue at a position corresponding to position 156 with alanine, substitution of an amino acid residue at a position corresponding to position 181 with lysine, substitution of an amino acid residue at a position corresponding to position 186 with glutamic acid, substitution of an amino acid residue at a position corresponding to position 188 with glutamine, substitution of an amino acid residue at a position corresponding to position 191 with valine, substitution of an amino acid residue at a position corresponding to position 220 with histidine, substitution of an amino acid residue at a position corresponding to position 221 with histidine, substitution of an amino acid residue at a position corresponding to position 224 with glutamine, substitution of an amino acid residue at a position corresponding to position 233 with threonine, substitution of an amino acid residue at a position corresponding to position 256 with asparagine, substitution of an amino acid residue at a position corresponding to position 260 with alanine, cysteine, or leucine, substitution of an amino acid residue at a position corresponding to position 269 with isoleucine, and substitution of an amino acid residue at a position corresponding to position 274 with glutamine numbered according to SEQ ID NO: 4. Any number of conservative amino acid substitutions may be performed as long as complex formation is not inhibited.

The polypeptide of the present invention can be isolated or purified in a monomeric or complex form by using general methods that are used in protein purification, for example centrifugation, ammonium sulfate precipitation, gel chromatography, ion exchange chromatography, and affinity chromatography, singly or in appropriate combination. The protein collected from the culture may be further purified by a known method.

### <8. Cleansing composition>

The polypeptide and the lipase complex of the present invention are useful as enzymes that are blended in various cleansing compositions and are particularly useful as enzymes that are blended in cleansing compositions suitable for low-temperature washing. Any one or both of the polypeptide and the lipase complex of the present invention are preferably used as an enzyme that is blended in a cleansing composition, and the lipase complex of the present invention is more preferably used as the enzyme, from the viewpoint of stability. Hereinafter, the polypeptide and the lipase complex of the present invention are collectively referred to as the polypeptide and the like of the present invention.

Here, the "low temperature" is, for example, 40°C or less, 35°C or less, 30°C or less, or 25°C or less and is, for example, 5°C or more, 10°C or more, or 15°C or more and is, for example, from 5°C to 40°C, from 10°C to 35°C, from 15°C to 30°C, or from 15°C to 25°C.

The amount of the polypeptide and the like of the present invention blended in the cleansing composition is not specifically limited as long as the polypeptide and the like exhibit the activity and is, based on 1 kg of the cleansing composition, for example, preferably 0.1 mg or more, more preferably 1 mg or more, and further preferably 5 mg or more and is preferably 5 000 mg or less, further preferably 1 000 mg or less, and further preferably 500 mg or less, and is preferably from 0.1 to 5 000 mg, more preferably from 1 to 1 000 mg, and further preferably from 5 to 500 mg.

The cleansing composition preferably includes a surfactant sulfosuccinic acid ester or a salt thereof, in addition to the polypeptide and the like of the present invention. The sulfosuccinic acid ester or a salt thereof is known as a component that is blended in cleansing compositions (for example, JP-A-2019-182911). The sulfosuccinic acid ester or a salt thereof is preferably a sulfosuccinic acid branched alkyl ester having a branched-chain alkyl group having 8 or more and 12 or less carbon atoms or a salt thereof, more preferably a sulfosuccinic acid branched alkyl ester having a branched-chain alkyl group having 9 or 10 carbon atoms or a salt thereof, and further preferably a sulfosuccinic acid branched alkyl ester having a branched-chain alkyl group having 10 carbon atoms or a salt thereof. The sulfosuccinic acid ester or a salt thereof is a sulfosuccinic acid branched dialkyl ester or a salt thereof and is preferably a sulfosuccinic acid branched dialkyl ester in which two branched-chain alkyl groups are each a branched-chain alkyl group having 8 or more and 12 or less carbon atoms or a salt thereof, more preferably a sulfosuccinic acid branched dialkyl ester in which two branched-chain alkyl groups are each a branched-chain alkyl group having 9 or 10 carbon atoms or a salt thereof, further preferably a sulfosuccinic acid branched dialkyl ester in which two branched-chain alkyl groups are each a branched-chain alkyl group having 10 carbon atoms or a salt thereof, and further preferably bis-(2-propylheptyl) sulfosuccinate or a salt thereof.

Examples of the salt include an alkali metal salt and an alkanolamine salt, and preferred is an alkali metal salt or an alkanolamine salt, more preferred is a salt selected from the group consisting of a sodium salt, a potassium salt, a triethanolamine salt, a diethanolamine salt, and a monoethanolamine salt, and further preferred is a sodium salt.

Examples of the sulfosuccinic acid ester or a salt thereof include compounds of the following Formula 1: 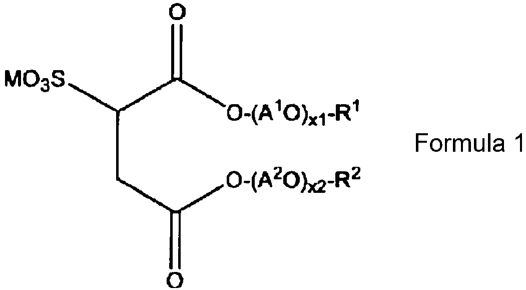 [in Formula 1, R¹ and R² are each a branched-chain alkyl group having 8 or more and 12 or less carbon atoms; A¹O and A²O are each an alkyleneoxy group having 2 or more and 4 or less carbon atoms; x1 and x2 are average addition mole numbers and are each a number of 0 or more and 10 or less; and M is a cation].

In Formula 1, R¹ and R² are each preferably a branched-chain alkyl group selected from the group consisting of a branched-chain nonyl group, a branched-chain decyl group, and a branched-chain dodecyl group and more preferably a branched-chain decyl group. The branched-chain decyl group is preferably a 2-propylheptyl group.

In Formula 1, A¹O and A²O are each an alkyleneoxy group having 2 or more and 4 or less carbon atoms and, preferably 2 or 3 carbon atoms from the viewpoint of lubricity to water. In Formula 1, x1 and x2 represent the average addition mole numbers of A¹O and A²O, respectively, and are each a number of 0 or more and 10 or less and, preferably 6 or less, more preferably 4 or less, and further preferably 2 or less, and are further preferably 0 from the viewpoint of lubricity to water.

In Formula 1, M is a cation. M is preferably a cation other than a hydrogen ion. Examples of M include an alkali metal ion such as a lithium ion, a sodium ion, and a potassium ion; an alkaline earth metal ion such as a calcium ion and a barium ion; and an organic ammonium ion such as a triethanolammonium ion, a diethanolammonium ion, a monoethanolammonium ion, a trimethylammonium ion, and a monomethylammonium ion.

M is preferably an alkali metal ion or an alkanolammonium ion, more preferably a sodium ion, a potassium ion, a triethanolammonium ion, a diethanolammonium ion, or a monoethanolammonium ion, and further preferably a sodium ion from the viewpoint of dispersibility in water.

The sulfosuccinic acid ester or a salt thereof is preferably a compound of the following Formula 1-1. The compound of Formula 1-1 is a compound with x1 and x2 in Formula 1 being each 0. 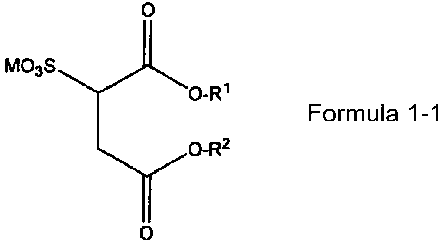 [in Formula 1-1, R¹ and R² are each a branched-chain alkyl group having 8 or more and 12 or less carbon atoms; and M is a cation].

Specific examples and preferable examples of R¹, R², and M in Formula 1-1 are the same as those in Formula 1.

In one preferable embodiment, the sulfosuccinic acid ester or a salt thereof is bis-(2-propylheptyl) sulfosuccinate or a salt thereof.

The amount of the sulfosuccinic acid ester or a salt thereof blended in the cleansing composition is preferably 0.01 mass% or more and more preferably 0.1 mass% or more and is preferably 30.0 mass% or less, more preferably 10.0 mass% or less, and further preferably 2.0 mass% or less. The amount is preferably from 0.01 to 30.0 mass%, more preferably from 0.1 to 10.0 mass%, and further preferably from 0.1 to 2.0 mass%.

The cleansing composition can include various enzymes in addition to the polypeptide and the like of the present invention, such as a hydrolase, an oxidase, a reductase, a transferase, a lyase, an isomerase, a ligase, and a synthetase. Among these enzymes, preferred are a lipase different from the lipase variant and the like of the present invention, an amylase, a protease, a cellulase, a keratinase, an esterase, a cutinase, a pullulanase, a pectinase, a mannanase, a glucosidase, a glucanase, a cholesterol oxidase, a peroxidase, and a laccase, and particularly preferred are a protease, a cellulase, an amylase, and a lipase different from the polypeptide of the present invention.

Examples of the protease include commercially available Alcalase, Esperase, Everlase, Savinase, Kannase, Progress Uno (registered trademark, Novozymes A/S), PREFERENZ, EFFECTENZ, EXCELLENZ (registered trademark, DuPont de Nemours, Inc.), Lavergy (registered trademark, BASF SE), and KAP (Kao Corporation). Alternative examples thereof include each variant of KP43 protease described in JP-A-2020-145938 and Japanese Patent Application No. 2022-118096.

Examples of the cellulase include Celluclean, Carezyme (registered trademark, Novozymes A/S), KAC, and an alkaline cellulase produced by *Bacillus* sp. KSM-S237 strain described in JP-A-10-313859 and a variant alkaline cellulase described in JP-A-2003-313592 (Kao Corporation).

Examples of the amylase include Termamyl, Duramyl, Stainzyme, Stainzyme Plus, Amplify Prime (registered trademark, Novozymes A/S), PREFERENZ, EFFECTENZ (registered trademark, DuPont de Nemours, Inc.), and KAM (Kao Corporation). Alternative examples thereof include each variant of YR288 amylase described in JP-A-2022-60158 and WO2023/176970. Alternative examples thereof include each variant of amylase described in JP-A-2019-500058 and JP-A-2018-516553.

Examples of the lipase include Lipolase and Lipex (registered trademark, Novozymes A/S).

The cleansing composition can include one or a combination of two or more of the enzymes described above, in addition to the polypeptide and the like of the present invention. Examples of the form of combined use include, but not limited to, combined use of the polypeptide and the like of the present invention and the protease, combined use of the polypeptide and the like of the present invention and the amylase, combined use of the polypeptide and the like of the present invention, the protease, and the amylase, combined use of the polypeptide and the like of the present invention, the lipase different from the polypeptide and the like of the present invention, and the protease, combined use of the polypeptide and the like of the present invention, the lipase different from the polypeptide and the like of the present invention, and the amylase, and combined use of the polypeptide and the like of the present invention, the lipase different from the polypeptide and the like of the present invention, the protease, and the amylase, among which combined use of the polypeptide and the like of the present invention, the protease, and the amylase is preferred.

The cleansing composition can include a known detergent component, and examples of the known detergent component include the following.

### (1) Surfactant

The surfactant is blended in an amount of from 0.5 to 90 mass% in the cleansing composition, in particular, preferably in an amount of from 10 to 45 mass% in a powder cleansing composition and in an amount of from 20 to 90 mass% in a liquid cleansing composition. When the cleansing composition is a laundry clothes detergent or an automatic dishwasher detergent, the surfactant is blended generally in an amount of from 0.5 to 20 mass%, preferably from 1 to 15 mass%, more preferably from 1.5 to 10 mass%, further more preferably from 2 to 8 mass%.

Examples of the surfactant that is used in the cleansing composition include, for example, one or a combination of an anionic surfactant, a nonionic surfactant other than the sulfosuccinic acid ester or a salt thereof, an amphoteric surfactant, and a cationic surfactant, and preferably an amphoteric surfactant.

Examples of the amphoteric surfactant include preferably an amine oxide surfactant or a betaine surfactant, and more preferably a tertiary amine oxide surfactant, sulfobetaine surfactant, or a carbobetaine surfactant. Examples of the tertiary amine oxide surfactant include a tertiary amine oxide surfactant in which one of the groups bound to the nitrogen atom is an alkyl group having 8 or more and 18 or less carbon atoms, preferably an alkyl group having 8 or more and 16 or less carbon atoms, further preferably an alkyl group having 8 or more and 14 or less carbon atoms, which may be interrupted by an amide group or an ester group, and the remaining group is an alkyl group having 1 or more and 3 or less carbon atoms and preferably a methyl group. Suitable examples of sulfobetaine surfactant include a compound having one alkyl group having 10 or more and 18 or less, preferably 16 or less, and more preferably 14 or less carbon atoms; two alkyl groups having 1 or more and 3 or less carbon atoms, preferably methyl groups; and a 3-sulfopropyl group or a 2-hydroxy-3-sulfopropyl group. Preferable examples of the carbobetaine surfactant include preferably a carbobetaine surfactant having one alkyl group having 10 or more and 18 or less, preferably 16 or less, and more preferably 14 or less carbon atoms, which may be interrupted by an amide group or an ester group; two alkyl groups having 1 or more and 3 or less carbon atoms, preferably methyl groups; and one carboxyalkyl group, preferably a carboxymethyl group.

### (2) Divalent metal ion scavenger

A divalent metal ion scavenger is blended in an amount of from 0.01 to 50 mass%, preferably from 5 to 40 mass%. Examples of the divalent metal ion scavenger that is used in the cleansing composition include a condensed phosphate such as a tripolyphosphate, a pyrophosphate, and an orthophosphate; an aluminosilicate such as zeolite; and a synthetic layered crystalline silicate, a nitrilotriacetate, an ethylenediaminetetraacetate, a citrate, an isocitrate, and a polyacetal carboxylate. Among these scavengers, a crystalline aluminosilicate (synthetic zeolite) is particularly preferable, and among A-type, X-type, and P-type zeolite, A-type is particularly preferable. Synthetic zeolite having an average primary particle diameter of from 0.1 to 10 µm, particularly, from 0.1 to 5 µm is suitably used.

### (3) Alkali agent

An alkali agent is blended in an amount of from 0.01 to 80 mass%, preferably from 1 to 40 mass%. Examples of the alkali agent that is used in a powder detergent include alkali metal carbonates such as sodium carbonate, collectively known as dense ash or light ash; and amorphous alkali metal silicates such as JIS Nos. 1, 2, and 3. These inorganic alkali agents are effective for particle skeleton formation during drying of the detergent and can provide a detergent that is relatively hard and has excellent fluidity. Other examples of the alkali agent include sodium sesquicarbonate and sodium hydrogen carbonate, and phosphates, such as tripolyphosphate, also have a function as an alkali agent. Examples of the alkali agent that is used in a liquid detergent include, in addition to the above-mentioned alkali agents, sodium hydroxide and mono-, di-, or tri-ethanolamine, and the alkali agent can also be used as a counterion of a surfactant.

### (4) Anti-resoiling agent

An anti-resoiling agent is blended in an amount of from 0.001 to 10 mass%, preferably from 1 to 5 mass%. Examples of the anti-resoiling agent that is used in the cleansing composition include polyethylene glycol, a carboxylic acid polymer, polyvinyl alcohol, and polyvinylpyrrolidone. Among these agents, the carboxylic acid polymer has, in addition to resoiling-preventing ability, a function of capturing metal ions and a function of dispersing solid particle soil from clothing into the wash bath. The carboxylic acid polymer is a homopolymer or a copolymer of acrylic acid, methacrylic acid, itaconic acid, or the like. A copolymer of the above-mentioned monomer and maleic acid is suitable, and the molecular weight is preferably from several thousands to 100 000. In addition to the above-mentioned carboxylic acid polymer, polymers such as polyglycidylate, cellulose derivatives such as carboxymethyl cellulose, and aminocarboxylic acid polymer such as polyaspartic acid are also preferable because of their metal ion scavenging, dispersing, and resoiling-preventing ability.

### (5) Bleaching agent

For example, a bleaching agent such as hydrogen peroxide and percarbonate is preferably blended in an amount of from 1 to 10 mass%. When a bleaching agent is used, tetraacetylethylenediamine (TAED) and a bleaching activator (activator) described in, for example, JP-A-6-316700 can be blended in an amount of from 0.01 to 10 mass%.

### (6) Fluorescent agent

Examples of a fluorescent agent that is used in the cleansing composition include a biphenyl fluorescent agent (e.g., Tinopal CBS-X) and a stilbene fluorescent agent (e.g., DM-type fluorescent dye). The fluorescent agent is preferably blended in an amount of from 0.001 to 2 mass%.

### (7) Chelating agent

The chelating agent is blended for, for example, easy washing of stains or reduction in water hardness upon washing.

Examples of the chelating agent include aminocarboxylic acid-based chelating agents, phosphonic acid-based chelating agents, hydroxycarboxylic acid-based chelating agents, and polyvalent carboxylic acid-based chelating agents.

Examples of the aminocarboxylic acid-based chelating agent include ethylenediaminetetraacetic acid (EDTA), nitrilotriacetic acid (NTA), hydroxyethylethylenediaminetriacetic acid (HEDTA), diethylenetriaminepentaacetic acid (DTPA), methylglycinediacetic acid (MGDA), triethylenetetraminehexaacetic acid (TTHA), glutamic acid diacetate (GLDA), hydroxyethyliminodiacetic acid (HIDA), dihydroxyethylglycine (DHEG), aspartic acid diacetate (ASDA), ethylenediaminesuccinic acid (EDDS), and their salts.

Examples of the phosphonic acid-based chelating agent include hydroxyethylidenediphosphonic acid (HEDP), nitrilotrismethylenephosphonic acid (NTMP), phosphonobutanetricarboxylic acid (PBTC), ethylenediaminetetramethylenephosphonic acid (EDTMP), and their salts.

Examples of the hydroxycarboxylic acid-based chelating agent include citric acid, malic acid, tartaric acid, gluconic acid, lactic acid, and their salts.

Examples of the polyvalent carboxylic acid-based chelating agent include succinic acid, oxalic acid, glutaric acid, adipic acid, fumaric acid, malonic acid, and their salts.

### (8) Other component

The cleansing composition can contain a solvent, a builder, a softener, a reducing agent (such as a sulfite), a foam inhibitor (such as silicone), a fragrance, an antibacterial and antifungal agent (Proxel [tradename], such as benzoic acid), and other additives that are known in the detergent field.

Examples of the solvent include a monovalent alcohol having 1 or more and 3 or less carbon atoms; a multivalent alcohol having 2 or more and 4 or less carbon atoms; a di- or tri-alkylene glycol of which the alkylene glycol unit has from 2 to 4 carbon atoms; and a monoalkoxy (methoxy, ethoxy, propoxy, or butoxy), phenoxy, or benzoxy ether of di- to tetra-alkylene glycols in which the alkylene glycol unit has from 2 to 4 carbon atoms.

The solvent is preferably a water-soluble organic solvent having 2 or more, preferably 3 or more, carbon atoms and having 10 or less, preferably 8 or less, carbon atoms. Here, the water-soluble organic solvent refers to a solvent having octanol/water partition coefficient (LogPow) of 3.5 or less.

Specifically, examples of the solvent include ethanol, isopropyl alcohol; ethylene glycol, propylene glycol, glycerin, isoprene glycol; diethylene glycol, dipropylene glycol; propylene glycol monomethyl ether, propylene glycol monoethyl ether, diethylene glycol monobutyl ether (also referred to as butyl diglycol), phenoxy ethanol, phenoxy triethylene glycol, and phenoxy isopropanol. The solvent is preferably selected from the group consisting of ethanol, propylene glycol, dipropylene glycol, diethylene glycol monobutyl ether, phenoxy ethanol, phenyl glycol, and phenoxy isopropanol. The solvent preferably includes an alkoxy group and further preferably includes at least one or more selected from monoalkoxy, phenoxy, or benzoxy ether of di- to tetra-alkylene glycol in which the alkylene glycol unit has from 2 to 4 carbon atoms. The solvent more preferably contains diethylene glycol monobutyl ether.

The cleansing composition preferably has a water content of from 60 to 99 mass% and/or a larger content of a solvent than that of a surfactant, more preferably a water content of from 60 to 99 mass% and a larger content of a solvent than that of a surfactant.

The cleansing composition can be produced by combining the polypeptide and the like of the present invention obtained by the above method and the above known washing components in accordance with a common method. The form of the detergent can be selected depending on the purpose and can be, for example, a liquid, a powder, a granule, a paste, or a solid.

The thus-obtained cleansing composition can be used as, for example, a clothes detergent, a dishwashing detergent, a bleaching agent, a detergent for washing hard surfaces, a drain pipe detergent, an artificial tooth detergent, or a bactericidal detergent for medical instruments, preferably a clothes detergent or a dishwashing detergent, and more preferably a laundry clothes detergent (laundry detergent), a dishwashing detergent for hand washing, or an automatic dishwasher detergent.

The cleansing composition is suitable for use at 40°C or less, 35°C or less, 30°C or less, or 25°C or less and at 5°C or more, 10°C or more, or 15°C or more and is suitable for use at from 5°C to 40°C, from 10°C to 35°C, from 15°C to 30°C, or from 15°C to 25°C. Examples of the preferable mode of use include use for low-temperature (from 15°C to 30°C) washing at laundries, use for low-temperature (from 15°C to 30°C) dishwashing by hand, and use for low-temperature (from 15°C to 30°C) washing by automatic dishwashers.

The use of the cleansing composition of the present invention enables washing an object to be washed (for example, clothing, tableware, hard surfaces, drain pipes, artificial teeth, and medical instruments) that require the removal of stains, i.e., enables removing stains. Such a washing method includes bringing an object to be washed that requires the removal of stains into contact with the cleansing composition of the present invention. Preferably, the stains are stains containing oil. Examples thereof include oil stains and combined stains containing proteins and carbohydrates in addition to oils.

In the washing method of the present invention, in order to bring an object to be washed and a cleansing composition into contact with each other, the object may be soaked in water in which the cleansing composition has been dissolved, or the cleansing composition may be directly applied to the object. In the method of the present invention, the object to be washed after soaking or application of a cleansing composition may be further hand washed, scrubbed with sponge, or washed with a washing machine, which is not necessarily needed.

Regarding the above-described embodiments, in the present invention, the following aspects are further disclosed.
<1> A lipase complex comprising two or more polypeptide molecules, wherein the two or more polypeptide molecules are connected via a disulfide bond, and at least one polypeptide molecule of the two or more polypeptide molecules is a polypeptide which consists of an amino acid sequence having an identity of at least 70% with the amino acid sequence of SEQ ID NO: 4, 6, 8, 10, 14, 16, 18, or 20 and has lipase activity.
<2> The lipase complex according to <1>, wherein the polypeptide having lipase activity consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4.
<3> The lipase complex according to <1> or <2>, wherein the lipase complex consists of two polypeptide molecules, wherein the two polypeptide molecules are connected via a disulfide bond, and the two polypeptide molecules are each independently a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<4> The lipase complex according to <1> or <2>, wherein the lipase complex consists of two identical polypeptide molecules, wherein the two polypeptide molecules are connected via a disulfide bond, and the polypeptide is a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<5> A method for producing a lipase complex, comprising the step of connecting, via a disulfide bond, two or more polypeptide molecules comprising at least one molecule of a polypeptide which consists of an amino acid sequence having an identity of at least 70% with the amino acid sequence of SEQ ID NO: 4, 6, 8, 10, 12, 14, 16 or 18 and has lipase activity.
<6> The method according to <5>, wherein the polypeptide having lipase activity consists of an amino acid sequence having an identity of at least 70% with the amino acid sequence of SEQ ID NO: 4, 6, 8, 10, 12, 14, 16, or 18 and having cysteine as an amino acid residue at one or more positions selected from the group consisting of positions corresponding to positions 208 and 272 numbered according to SEQ ID NO: 4.
<7> The method according to <5> or <6>, wherein the connection step comprises connecting two polypeptide molecules via a disulfide bond, wherein the two polypeptide molecules are each independently a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<8> The method according to <5> or <6>, wherein the connection step comprises connecting two identical polypeptide molecules via a disulfide bond, wherein the polypeptide is a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<9> The method according to any one of <5> to <8>, further comprising, before the connection step, the step of providing the polypeptide which consists of an amino acid sequence having an identity of at least 70% with the amino acid sequence of SEQ ID NO: 4, 6, 8, 10, 12, 14, 16, or 18 and having cysteine as an amino acid residue at one or more positions selected from the group consisting of positions corresponding to positions 208 and 272 numbered according to SEQ ID NO: 4, and has lipase activity.
<10> A method for producing a lipase complex, comprising the steps of:
   substituting, with cysteine, an amino acid residue at one or more positions selected from the group consisting of positions corresponding to positions 208 and 272 numbered according to SEQ ID NO: 4 in a polypeptide which consists of an amino acid sequence having an identity of at least 70% with the amino acid sequence of SEQ ID NO: 4, 6, 8, 10, 12, 14, 16, or 18 and has lipase activity; and
   connecting, via a disulfide bond, two or more polypeptide molecules comprising at least one molecule of a polypeptide after amino acid residue substitution.
<11> The method according to <10>, wherein the connection step comprises connecting two polypeptide molecules after amino acid residue substitution via a disulfide bond, wherein the two polypeptide molecules after amino acid residue substitution are each independently a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<12> The method according to <10>, wherein the connection step comprises connecting two identical polypeptide molecules after amino acid residue substitution via a disulfide bond.
<13> The method according to any one of <5> to <12>, wherein the connection step is performed under a non-reducing condition, and the connection step is preferably performed by expressing or retaining the polypeptides under a non-reducing condition.
<14> A method for improving stability of a lipase, comprising the step of performing lipase complexation.
<15> The method according to <14>, comprising the step of connecting, via a disulfide bond, two or more polypeptide molecules comprising at least one molecule of a polypeptide which consists of an amino acid sequence having an identity of at least 70% with the amino acid sequence of SEQ ID NO: 4, 6, 8, 10, 12, 14, 16 or 18 and has lipase activity.
<16> The method according to <15>, wherein the polypeptide having lipase activity consists of an amino acid sequence having an identity of at least 70% with the amino acid sequence of SEQ ID NO: 4, 6, 8, 10, 12, 14, 16, or 18 and having cysteine as an amino acid residue at one or more positions selected from the group consisting of positions corresponding to positions 208 and 272 numbered according to SEQ ID NO: 4.
<17> The method according to <15> or <16>, wherein the connection step comprises connecting two polypeptide molecules via a disulfide bond, wherein the two polypeptide molecules are each independently a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<18> The method according to <15> or <16>, wherein the connection step comprises connecting two identical polypeptide molecules via a disulfide bond, wherein the polypeptide is a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<19> The method according to any one of <15> to <18>, further comprising, before the connection step, the step of providing the polypeptide which consists of an amino acid sequence having an identity of at least 70% with the amino acid sequence of SEQ ID NO: 4, 6, 8, 10, 12, 14, 16, or 18 and having cysteine as an amino acid residue at one or more positions selected from the group consisting of positions corresponding to positions 208 and 272 numbered according to SEQ ID NO: 4, and has lipase activity.
<20> A method for improving stability of a lipase, comprising the steps of:
   substituting, with cysteine, an amino acid residue at one or more positions selected from the group consisting of positions corresponding to positions 208 and 272 numbered according to SEQ ID NO: 4 in a polypeptide which consists of an amino acid sequence having an identity of at least 70% with the amino acid sequence of SEQ ID NO: 4, 6, 8, 10, 12, 14, 16, or 18 and has lipase activity; and
   connecting, via a disulfide bond, two or more polypeptide molecules comprising at least one molecule of a polypeptide after amino acid residue substitution.
<21> The method according to <20>, wherein the connection step comprises connecting two polypeptide molecules after amino acid residue substitution via a disulfide bond, wherein the two polypeptide molecules after amino acid residue substitution are each independently a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<22> The method according to <20>, wherein the connection step comprises connecting two identical polypeptide molecules after amino acid residue substitution via a disulfide bond.
<23> The method according to any one of <15> to <22>, wherein the connection step is performed under a non-reducing condition, and the connection step is preferably performed by expressing or retaining the polypeptides under a non-reducing condition.
<24> A polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.
<25> The complex according to any one of <1> to <4> or the polypeptide according to <24>, wherein the complex or the polypeptide has one or more amino acid residues selected from the group consisting of phenylalanine at a position corresponding to position 16, cysteine or methionine at a position corresponding to position 22, phenylalanine at a position corresponding to position 29, isoleucine or valine at a position corresponding to position 50, alanine or glutamine at a position corresponding to position 120, isoleucine or threonine at a position corresponding to position 123, glutamic acid at a position corresponding to position 126, phenylalanine at a position corresponding to position 142, alanine at a position corresponding to position 156, lysine at a position corresponding to position 181, glutamic acid at a position corresponding to position 186, glutamine at a position corresponding to position 188, valine at a position corresponding to position 191, histidine at a position corresponding to position 220, histidine at a position corresponding to position 221, glutamine at a position corresponding to position 224, threonine at a position corresponding to position 233, asparagine at a position corresponding to position 256, alanine, cysteine, or leucine at a position corresponding to position 260, isoleucine at a position corresponding to position 269, and glutamine at a position corresponding to position 274 numbered according to SEQ ID NO: 4.
<26> The complex according to any one of <1> to <4> and <25> or the polypeptide according to <24> or <25>, wherein the polypeptide having lipase activity is a variant polypeptide.
<27> A polynucleotide encoding the polypeptide according to any one of <24> to <26>.
<28> A vector or DNA fragment comprising the polynucleotide according to <27>.
<29> A transformed cell comprising the vector or DNA fragment according to <28>.
<30> The transformed cell according to <29>, wherein the transformed cell is a microorganism.
<31> The transformed cell according to <29>, wherein the transformed cell is *Escherichia coli* or a *Bacillus* bacterium, preferably a *Bacillus* bacterium, and more preferably *Bacillus subtilis.*
<32> A method for producing a polypeptide having lipase activity, comprising the step of culturing the transformed cell according to any one of <29> to <31>.
<33> A cleansing composition comprising any one or both of the lipase complex according to any one of <1> to <4>, <25>, and <26> and the polypeptide according to any one of <24> to <26>, preferably the lipase complex according to any one of <1> to <4>, <25>, and <26>, or both of the lipase complex according to any one of <1> to <4>, <25>, and <26> and the polypeptide according to any one of <24> to <26>.
<34> The cleansing composition according to <33>, further comprising a sulfosuccinic acid ester or a salt thereof, preferably a sulfosuccinic acid branched alkyl ester having a branched-chain alkyl group having 8 or more and 12 or less carbon atoms or a salt thereof, more preferably a sulfosuccinic acid branched alkyl ester having a branched-chain alkyl group having 9 or 10 carbon atoms or a salt thereof, and further preferably a sulfosuccinic acid branched alkyl ester having a branched-chain alkyl group having 10 carbon atoms or a salt thereof.
<35> The cleansing composition according to <33>, further comprising a sulfosuccinic acid diester or a salt thereof, preferably a sulfosuccinic acid branched dialkyl ester in which two branched-chain alkyl groups are each a branched-chain alkyl group having 8 or more and 12 or less carbon atoms or a salt thereof, more preferably a sulfosuccinic acid branched dialkyl ester in which two branched-chain alkyl groups are each a branched-chain alkyl group having 9 or 10 carbon atoms or a salt thereof, further preferably a sulfosuccinic acid branched dialkyl ester in which two branched-chain alkyl groups are each a branched-chain alkyl group having 10 carbon atoms or a salt thereof, and further preferably bis-(2-propylheptyl) sulfosuccinate or a salt thereof.
<36> The cleansing composition according to any one of <33> to <35>, wherein a content of water is from 60 to 99 mass%, and/or a content of a solvent is larger than that of a surfactant, and preferably, a content of water is from 60 to 99 mass%, and a content of a solvent is larger than that of a surfactant.
<37> The cleansing composition according to any one of <33> to <36> which is a clothes detergent or a dishwashing detergent.
<38> The cleansing composition according to any one of <33> to <37> which is a powder or a liquid.
<39> A stain-washing method comprising using the cleansing composition according to any one of <33> to <38>.
<40> The method according to <39>, comprising bringing an object to be washed into contact with the cleansing composition according to any one of <33> to <38>.
<41> Use of any one or both of the lipase complex according to any one of <1> to <4>, <25>, and <26> and the polypeptide according to any one of <24> to <26>, preferably the lipase complex according to any one of <1> to <4>, <25>, and <26>, or both of the lipase complex according to any one of <1> to <4>, <25>, and <26> and the polypeptide according to any one of <24> to <26> for producing a cleansing composition.
<42> Use of any one or both of the lipase complex according to any one of <1> to <4>, <25>, and <26> and the polypeptide according to any one of <24> to <26>, preferably the lipase complex according to any one of <1> to <4>, <25>, and <26>, or both of the lipase complex according to any one of <1> to <4>, <25>, and <26> and the polypeptide according to any one of <24> to <26> for washing a stain.

### Examples

The present invention will be described in further detail based on Examples below, but is not limited thereto.

### Example 1

### (1) Construction of lipase expression plasmid

A plasmid pHY-CnLipA was constructed by using a plasmid pHY-S237 described in Example 7 of WO 2006/068148A1 as a template and transferring an artificially synthesized gene of CnLip I44T L120S S130A A134T A135V L136M S137N (hereinafter, referred to as CnLipA; the polynucleotide of SEQ ID NO: 1 which encodes the amino acid sequence of SEQ ID NO: 2) described in Japanese Patent Application No. 2023-025141 to a full-length ORF of an alkaline cellulase gene by In-Fusion reaction. A plasmid pHY-CnLipAm for expression of CnLipAm (the polynucleotide of SEQ ID NO: 3 which encodes the amino acid sequence of SEQ ID NO: 4) was prepared by using pHY-CnLipA as a template and introducing thereto five mutations by site-directed mutagenesis by PCR using a complementary primer pair (Zheng, Lei, Ulrich Baumann, and Jean-Louis Reymond. Nucleic Acids Research 32.14 (2004): e115).

Plasmids pHY-SspLip_m, pHY-EtLip_m, and pHY-CspLipA were constructed by using a plasmid pHY-S237 described in Example 7 of WO 2006/068148A1 as a template and transferring an artificially synthesized gene of SspLip_m (the polynucleotide of SEQ ID NO: 5 which encodes the amino acid sequence of SEQ ID NO: 6), EtLip_m (the polynucleotide of SEQ ID NO: 7 which encodes the amino acid sequence of SEQ ID NO: 8), or CspLip I45T L121S S131A A135T A136V S138N A213M (hereinafter, referred to as CspLipA; the polynucleotide of SEQ ID NO: 9 which encodes the amino acid sequence of SEQ ID NO: 10) to a full-length ORF of an alkaline cellulase gene by In-Fusion reaction.

In further mutagenesis to each lipase, the site-directed mutagenesis by PCR using a complementary primer pair was used.

### (2) Preparation of lipase solution

A lipase expression plasmid was introduced into a *Bacillus subtilis* strain 168ΔsigF described in JP-A-2003-47490 by a protoplast method. The strain was cultured in 2 × L-maltose culture medium (2% tryptone, 1% yeast extract, 1% NaCl, 7.5% maltose, 7.5 ppm manganese sulfate pentahydrate, 0.04% calcium chloride dihydrate, and 15 ppm tetracycline: % is (w/v)%) at 30°C for 4 days, and the culture supernatant containing the lipase was then collected by centrifugation.

### (3) SDS-PAGE of lipase variant

Each culture supernatant containing CnLipAm (parent enzyme), CnLipAm L208C variant, or CnLipAm N272C variant prepared by the method described in the section (2) was mixed with Laemmli Sample Buffer (Bio-Rad Laboratories, Inc.) containing or not containing 100 mM DTT as a reducing agent, in equal amounts and then heat-treated at 99°C for 5 minutes to prepare a sample. The gel used was Mini-PROTEIN TGX Stain-Free (Bio-Rad Laboratories, Inc.). 5 µL of the sample was applied to each well and migrated at 210 V for 25 minutes. The molecular weight marker used was Precision Plus protein Unstained standard (Bio-Rad Laboratories, Inc.). Protein band intensity was calculated with ChemiDoc MP Imaging System. A monomer ratio (%) was determined by dividing the band intensity in a non-reduced state of each variant by the band intensity in a reduced state (Table 1). The monomer ratio was decreased in the variants, revealing that a complex was formed via disulfide formation in the culture supernatant.

**[Table 1]**

| Lipase | Monomer ratio (%) |
|---|---|
| CnLipAm (parent enzyme) | 106.6 |
| L208C | 68.8 |
| N272C | 88.0 |

### (4) Purification of lipase complex by gel filtration chromatography

Each culture supernatant containing CnLipAm (parent enzyme), CnLipAm L208C variant, or CnLipAm N272C variant prepared by the method described in the section (2) was applied to Amicon Ultra 10K (Merck Millipore), and the buffer was replaced with 20 mM Tris-HCl (pH 7.0), 2 mM CaCl₂, 0.01% Triton-X100, and 150 mM NaCl to prepare a lipase sample for gel filtration. The lipase sample for gel filtration was subjected to gel filtration chromatography under the following conditions to give fractionated samples.

### (Conditions)

Apparatus: AKTA pure (GE Healthcare)
Column: HiLoad 26/600 Superdex 200pg (Cytiva)
Buffer: 20 mM Tris-HCl (pH 7.0), 2 mM CaCl₂, 0.01%
Triton-X100, and 150 mM NaCl
Flow rate: 1 mL/min
Elute fraction size: 1.5 mL
Length of elution: 1.3 CV

4-Nitrophenyl butyrate (SIGMA) was used as a substrate in lipase activity measurement of the fractionated samples. The activity of a lipase can be determined by measuring the rate of increase in absorbance due to release of 4-nitrophenol by the function of a lipase. A mixture of 4-nitrophenyl butyrate added at a final concentration of 2 mM to 20 mM Tris-HCl (pH 7.0) was used as a substrate solution. Activity measurement was performed using the fractionated samples diluted 51-fold with 20 mM Tris-HCl (pH 7.0), and fractions containing the CnLipAm L208C monomer, the CnLipAm L208C complex 1, the CnLipAm L208C complex 2, the CnLipAm N272C monomer, the CnLipAm N272C complex 1, the CnLipAm N272C complex 2, and the CnLipAm (parent enzyme) monomer, respectively, were identified by using lipase activity as an indicator.

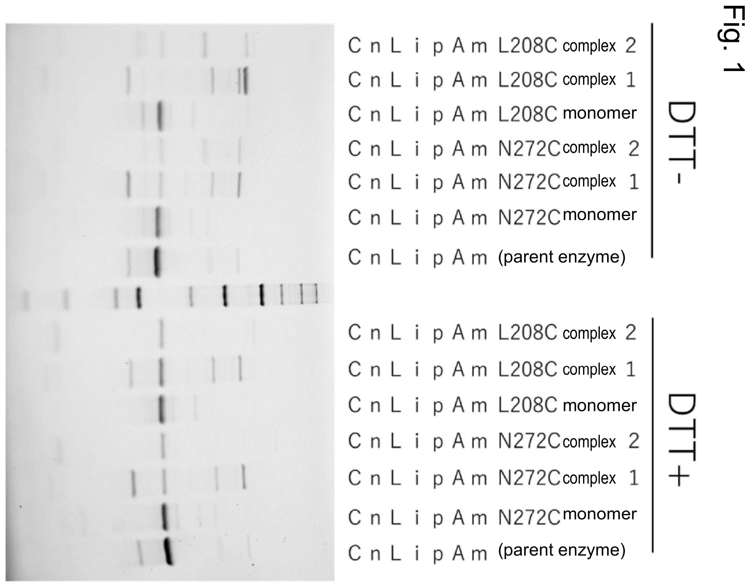

Each fraction solution containing the lipase monomer or complex was subjected to SDS-PAGE by the method described in the section (3) (Figure 1). The CnLipAm L208C complex 1 was found as a fraction containing a homodimer from a clear band confirmed at a molecular weight of twice the putative molecular weight. The CnLipAm N272C complex 2 contained a homodimer from a band confirmed at a molecular weight of twice the putative molecular weight, and was further confirmed to contain a complex via a disulfide bond with a component contained in the culture supernatant. The CnLipAm L208C complex 2 and the CnLipAm N272C complex 1 were each confirmed to be a complex formed via a disulfide bond with a component contained in the culture supernatant.

### (5) Evaluation of stability in model washing solution

4-Nitrophenyl butyrate (SIGMA) was used as a substrate. The activity of a lipase can be determined by measuring the rate of increase in absorbance due to release of 4-nitrophenol by the function of a lipase. A mixture of 4-nitrophenyl butyrate added at a final concentration of 2 mM to 20 mM Tris-HCl (pH 7.0) was used as a substrate solution.

Each fraction solution containing the lipase monomer or complex was added to the model washing solution shown in Table 2, then incubated at 45°C for 1 hour, and then diluted 51-fold with 20 mM Tris-HCl (pH 7.0), and each dilution was used in activity measurement. A deactivation rate per unit time (h) by the treatment at 45°C was calculated with the activity value of the sample before the treatment at 45°C as initial activity, and a half-life (h) was calculated therefrom. Relative stability was determined by dividing the half-life (h) of each variant by the half-life (h) of the parent polypeptide. The results are shown in Table 3. The CnLipAm L208C and CnLipAm N272C variants were found to be drastically stabilized by complex formation compared to the parent enzyme.

**[Table 2]**

| Blended component | mass% |
|---|---|
| Sulfosuccinic acid branched alkyl ester | 0.2 |
| Betaine | 2 |
| Butyl diglycol | 6.5 |
| Citric acid | 2 |
| NaOH | Added so as to attain pH 6.5 |
| Water | Balance |
| Total | 100 |

| | |
|---|---|
| · Sulfosuccinic acid branched alkyl ester: sodium bis-(2-propylheptyl)sulfosuccinate · Betaine: lauryl (2-hydroxy-3-sulfopropyl)dimethylbetaine, AMPHITOL 20HD, manufactured by Kao Corp. | |

**[Table 3]**

| Lipase solution | Relative stability |
|---|---|
| CnLipAm L208C complex 2 | 3.24 |
| CnLipAm L208C complex 1 | 4.77 |
| CnLipAm L208C monomer | 1.70 |
| CnLipAm N272C complex 2 | 6.66 |
| CnLipAm N272C complex 1 | 3.37 |
| CnLipAm N272C monomer | 2.03 |
| CnLipAm (parent enzyme) monomer | 1.00 |

### (6) Evaluation of detergency in model washing solution

The ability to remove triglycerides on a hard surface was evaluated as detergency. Beef tallow (SIGMA, 03-0660) and rapeseed oil (SIGMA, 23-0450) were mixed at a weight ratio of 9 : 1, the mixture was dissolved in triple volumes of chloroform, and the solution was then colored with 0.2 wt% Sudan III to give a model stain. Ten microliter of the model stain was dropwise added to each well bottom of a 96-well polypropylene deep well plate, and the chloroform was evaporated and dried to make a stain plate.

The fraction obtained by gel filtration chromatography in the section (4) was diluted with 20 mM Tris-HCl (pH 7.0), 2 mM CaCl₂, 0.01% Triton-X100, and 150 mM NaCl to a lipase concentration of 200 mg/L, and the resulting lipase solution was added to the model washing solution described in Table 2 in an amount of 1/25 of the model washing solution. Immersion washing was performed by gently adding 300 µL of the washing solution to each well of the stain plate and leaving it to stand at room temperature (about 22°C) for 20 minutes. One hundred microliter of each washing solution was taken from each well while avoiding contact with the stain on the bottom and was transferred to another 96-well plate. In order to quantitatively measure Sudan III in a model stain that was solubilized in the washing solution by the immersion washing, the absorbance (A500) at 500 nm was measured. A500 corresponds to the release amount of oil into the washing solution and can be used as an indicator of detergency. The enzymatic effect (ΔA500) on detergency was determined by subtracting A500 of a washing solution containing 20 mM Tris-HCl (pH 7.0), 2 mM CaCl₂, 0.01% Triton-X100, and 150 mM NaCl instead of the lipase from A500 of the washing solution containing each lipase. The results of washing for 20 minutes are shown in Table 4. The CnLipAm L208C and CnLipAm N272C variants were found to maintain detergency even after complex formation.

**[Table 4]**

| Lipase solution | ΔA500 |
|---|---|
| CnLipAm L208C dimer 2 | 0.101 |
| CnLiPAm L208C dimer 1 | 0.088 |
| CnLiPAm L208C monomer | 0.097 |
| CnLiPAm N272C dimer 2 | 0.076 |
| CnLiPAm N272C dimer 1 | 0.090 |
| CnLiPAm N272C monomer | 0.089 |
| CnLiPAm (parent enzyme) monomer | 0.073 |

### (7) Evaluation of stability of lipase variant

4-Nitrophenyl butyrate (SIGMA) was used as a substrate. The activity of a lipase can be determined by measuring the rate of increase in absorbance due to release of 4-nitrophenol by the function of a lipase. A mixture of 4-nitrophenyl butyrate added at a final concentration of 2 mM to 20 mM Tris-HCl (pH 7.0) was used as a substrate solution.

Each culture supernatant containing SspLip_m, EtLip_m, CspLipA, or SspLip_m L210C, EtLip_m F210C, or CspLipA L209C variant prepared by the method described in the section (2) was added to the model washing solution shown in Table 2, then incubated at 40°C or 45°C for an optional time, and then diluted 51-fold with 20 mM Tris-HCl (pH 7.0), and each dilution was used in activity measurement. A deactivation rate per unit time (h) by the heat treatment was calculated with the activity value of the sample before the heat treatment as initial activity, and a half-life (h) was calculated therefrom. Relative stability was determined by dividing the half-life (h) of each variant by the half-life (h) of the parent polypeptide. The results are shown in Table 5. All the variants were found to have improved stability compared to the parent polypeptide. Each of position 210 of SspLip_m, position 210 of EtLip_m, and position 209 of CspLipA corresponds to position 208 of CnLipAm.

**[Table 5]**

| Lipase solution | Relative stability | Heat treatment conditions |
|---|---|---|
| SspLip_m L210C | 2.48 | 40°C, 45 min |
| EtLip_m F210C | 1.55 | 40°C, 45 min |
| CspLipA L209C | 2.38 | 45°C, 50 min |

### (8) SDS-PAGE of lipase variant

Each culture supernatant containing SspLip_m, EtLip_m, CspLipA, or SspLip_m L210C, EtLip_m F210C, or CspLipA L209C variant prepared by the method described in the section (2) was mixed with Laemmli Sample Buffer (Bio-Rad Laboratories, Inc.) containing or not containing 100 mM DTT as a reducing agent, in equal amounts and then heat-treated at 99°C for 5 minutes to prepare a sample. The gel used was Mini-PROTEIN TGX Stain-Free (Bio-Rad Laboratories, Inc.). 5 µL of the sample was applied to each well and migrated at 210 V for 25 minutes. The molecular weight marker used was Precision Plus protein Unstained standard (Bio-Rad Laboratories, Inc.). Protein band intensity was calculated with ChemiDoc MP Imaging System. A monomer ratio (%) was determined by dividing the band intensity in a non-reduced state of each variant by the band intensity in a reduced state (Table 6). The monomer ratio was less than 100% in the variants, revealing that a complex was formed via disulfide formation in the culture supernatant.

**[Table 6]**

| Lipase | Monomer ratio (%) |
|---|---|
| SspLip_m L210C | 61.8 |
| EtLip_m F210C | 46.8 |
| CspLipA L209C | 61.8 |

### Example 2: Evaluation of stability of combination variant

4-Nitrophenyl butyrate (SIGMA) was used as a substrate. The activity of a lipase can be determined by measuring the rate of increase in absorbance due to release of 4-nitrophenol by the function of a lipase. A mixture of 4-nitrophenyl butyrate added at a final concentration of 2 mM to 20 mM Tris-HCl (pH 7.0) was used as a substrate solution.

Each lipase solution was added to the model washing solution shown in Table 2, then incubated at 40°C for 3 hours, and then diluted 51-fold with 20 mM Tris-HCl (pH 7.0), and each dilution was used in activity measurement. A deactivation rate per unit time (h) by the treatment at 40°C was calculated with the activity value of the sample before the treatment at 40°C as initial activity, and a half-life (h) was calculated therefrom. Relative stability was determined by dividing the half-life (h) of each variant by the half-life (h) of the parent polypeptide. The results are shown in Table 7. All the variants were found to have improved stability compared to the parent polypeptide.

**[Table 7-1]**

| (Relative stability in treatment at 40°C for 3 hr) | | | | |
|---|---|---|---|---|
| Lipase | Relative stability | | Lipase | Relative stability |
| CnLipAm N272C (parent enzyme) | 1.00 | | V260A | 2.17 |
| Y29F | 1.15 | | V260C | 4.95 |
| L142F | 1.32 | | V260L | 1.81 |
| V220H | 1.72 | | V2691 | 2.34 |
| T274Q | 1.31 | | A221H | 2.78 |
| K1231 | 1.41 | | L16F | 1.30 |
| A224Q | 1.43 | | G22M | 3.75 |
| K123T | 1.09 | | L501 | 1.23 |
| A181K | 1.10 | | L50V | 1.22 |
| D191V | 1.27 | | G156A | 1.52 |
| L208C | 2.25 | | S120A | 1.15 |
| Q186E | 1.08 | | S120Q | 1.36 |
| K188Q | 1.45 | | R233T | 2.08 |
| R126E | 1.22 | | S256N | 3.26 |

**[Table 7-2]**

| (Relative stability in treatment at 40°C for 3 hr) | |
|---|---|
| Lipase | Relative stability |
| CnLipAm L208C (parent enzyme) | 1.00 |
| V220H | 3.26 |
| A224Q | 1.21 |
| K188Q | 1.50 |
| R126E | 1.15 |
| N272G | 3.50 |
| V260A | 1.65 |
| V260C | 1.81 |
| V260L | 1.82 |
| V2691 | 2.13 |
| L16F | 1.54 |
| L501 | 1.02 |
| G22C | 2.15 |
| S256N | 5.27 |

### Reference Example 1: Comparison of stability between model washing solution and another formulation

A commercially available liquid detergent (Kao Corp., Attack ZERO (hereinafter, referred to as detergent 1) was left to stand for 30 minutes in a boiling water bath to deactivate the blended enzyme. Savinase (SIGMA, P3111) was added at a final concentration of 0.5% to the detergent and thoroughly mixed. CnLipAm prepared by the method described in Example 1(2) was added to the model washing solution shown in Table 2 and the detergent 1 containing 0.5% Savinase, then incubated at 45°C for 1 hour, and then diluted 51-fold with 20 mM Tris-HCl (pH 7.0), and each dilution was used in activity measurement. The activity value of the sample before the treatment at 45°C was defined as initial activity, and the ratio of the activity value after the treatment to the initial activity was used as residual activity (%). The results are shown in Table 8. A phenomenon was confirmed in which the lipase was significantly destabilized in the model washing solution described in Table 2 whose suitability for washing was disclosed in Patent Literature 1, even compared to another formulation containing a protease which degrades proteins.

**[Table 8]**

| Formulation | Residual activity (%) |
|---|---|
| Model formulation of Table 2 | 3.61 |
| Detergent 1 + 0.5% Savinase | 67.75 |

### Reference Example 2: Evaluation of stability of lipase variant

4-Nitrophenyl butyrate (SIGMA) was used as a substrate. The activity of a lipase can be determined by measuring the rate of increase in absorbance due to release of 4-nitrophenol by the function of a lipase. A mixture of 4-nitrophenyl butyrate added at a final concentration of 2 mM to 20 mM Tris-HCl (pH 7.0) was used as a substrate solution.

A plasmid pHY-PvLip was constructed by using a plasmid pHY-S237 described in Example 7 of WO 2006/068148A1 as a template and transferring an artificially synthesized gene of PvLip (the polynucleotide of SEQ ID NO: 11 which encodes the amino acid sequence of SEQ ID NO: 12) to a full-length ORF of an alkaline cellulase gene by In-Fusion reaction. In further mutagenesis to the lipase, the site-directed mutagenesis by PCR using a complementary primer pair was used.

Each culture supernatant containing PvLip or PvLip A205C variant prepared by the method described in Example 1(2) was added to the model washing solution shown in Table 2, then incubated at 45°C for 50 minutes, and then diluted 51-fold with 20 mM Tris-HCl (pH 7.0), and each dilution was used in activity measurement. A deactivation rate per unit time (h) by the heat treatment was calculated with the activity value of the sample before the heat treatment as initial activity, and a half-life (h) was calculated therefrom. Relative stability was determined by dividing the half-life (h) of each variant by the half-life (h) of the parent polypeptide. The results are shown in Table 9. The PvLip A205C variant was found to have no improved stability compared to the parent polypeptide. Position 205 of PvLip corresponds to position 208 of CnLipAm.

**[Table 9]**

| Lipase solution | Relative stability |
|---|---|
| PvLip A205C | 0.92 |

### Reference Example 3: SDS-PAGE of lipase variant

Each culture supernatant containing PvLip or PvLip A205C variant prepared by the method described in Example 1(2) was mixed with Laemmli Sample Buffer (Bio-Rad Laboratories, Inc.) containing or not containing 100 mM DTT as a reducing agent, in equal amounts and then heat-treated at 99°C for 5 minutes to prepare a sample. The gel used was Mini-PROTEIN TGX Stain-Free (Bio-Rad Laboratories, Inc.). 5 µL of the sample was applied to each well and migrated at 210 V for 25 minutes. The molecular weight marker used was Precision Plus protein Unstained standard (Bio-Rad Laboratories, Inc.). Protein band intensity was calculated with ChemiDoc MP Imaging System. A monomer ratio (%) was determined by dividing the band intensity in a non-reduced state of each variant by the band intensity in a reduced state (Table 10). The monomer ratio was 100% in the PvLip A205C variant, revealing that no complex was formed via disulfide formation in the culture supernatant.

**[Table 10]**

| Lipase | Monomer ratio (%) |
|---|---|
| PvLip A205C | 102.2 |

### Reference Example 4: Evaluation of stability of lipase variant

4-Nitrophenyl butyrate (SIGMA) was used as a substrate. The activity of a lipase can be determined by measuring the rate of increase in absorbance due to release of 4-nitrophenol by the function of a lipase. A mixture of 4-nitrophenyl butyrate added at a final concentration of 2 mM to 20 mM Tris-HCl (pH 7.0) was used as a substrate solution.

Each culture supernatant containing CnLipAm (parent enzyme), CnLipAm P207C variant, or CnLipAm H209C variant prepared by the method described in Example 1(2) was added to the model washing solution shown in Table 2, then incubated at 40°C for 1 hour, and then diluted 51-fold with 20 mM Tris-HCl (pH 7.0), and each dilution was used in activity measurement. A deactivation rate per unit time (h) by the heat treatment was calculated with the activity value of the sample before the heat treatment as initial activity, and a half-life (h) was calculated therefrom. Relative stability was determined by dividing the half-life (h) of each variant by the half-life (h) of the parent polypeptide. The results are shown in Table 11. All the variants were confirmed to have no improved stability compared to the parent polypeptide.

**[Table 11]**

| Lipase | Relative stability |
|---|---|
| CnLipAm (parent enzyme) | 1.00 |
| H209C | 0.84 |
| P207C | 1.00 |

## Claims

1. A lipase complex comprising two or more polypeptide molecules, wherein the two or more polypeptide molecules are connected via a disulfide bond, and at least one polypeptide molecule of the two or more polypeptide molecules is a polypeptide which consists of an amino acid sequence having an identity of at least 70% with the amino acid sequence of SEQ ID NO: 4, 6, 8, 10, 14, 16, 18, or 20 and has lipase activity.

2. The lipase complex according to claim 1, wherein the polypeptide having lipase activity consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4.

3. The lipase complex according to claim 1, wherein the lipase complex consists of two polypeptide molecules, wherein the two polypeptide molecules are connected via a disulfide bond, and the two polypeptide molecules are each independently a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.

4. A method for producing a lipase complex, comprising the step of connecting, via a disulfide bond, two or more polypeptide molecules comprising at least one molecule of a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.

5. The method according to claim 4, further comprising, before the connection step, the step of providing the polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.

6. A method for improving stability of a lipase, comprising the step of connecting, via a disulfide bond, two or more polypeptide molecules comprising at least one molecule of a polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.

7. The method according to claim 6, further comprising, before the connection step, the step of providing the polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.

8. A polypeptide which consists of an amino acid sequence having an identity of at least 70% with the amino acid sequence of SEQ ID NO: 4, 6, 8, 10, 14, 16, 18, or 20 and having cysteine as an amino acid residue at one or more positions selected from the group consisting of positions corresponding to positions 208 and 272 numbered according to SEQ ID NO: 4, and has lipase activity.

9. A polynucleotide encoding the polypeptide according to claim 8.

10. A vector or DNA fragment comprising the polynucleotide according to claim 9.

11. A transformed cell comprising the vector or DNA fragment according to claim 10.

12. The transformed cell according to claim 11, wherein the transformed cell is a microorganism.

13. A cleansing composition comprising one or both of the lipase complex according to any one of claims 1 to 3 and the polypeptide according to claim 8.
